(19) Europäisches Patentamt
European Patent Office
Office européen des brevets

(11) **EP 4 379 043 A1**

(12) **EUROPEAN PATENT APPLICATION**
published in accordance with Art. 153(4) EPC

(43) Date of publication:
**05.06.2024 Bulletin 2024/23**

(21) Application number: 22849909.1

(22) Date of filing: **28.07.2022**

(51) International Patent Classification (IPC):
*C12N 5/0775* (2010.01)     *C07K 14/705* (2006.01)
*C12N 15/10* (2006.01)      *C12N 15/113* (2010.01)
*C12N 15/90* (2006.01)      *C12N 9/22* (2006.01)
*A61K 48/00* (2006.01)      *A61K 35/28* (2015.01)

(52) Cooperative Patent Classification (CPC):
**A61K 35/28; A61K 48/00; C07K 14/705;
C12N 5/06; C12N 9/22; C12N 15/10; C12N 15/113;
C12N 15/90**

(86) International application number:
**PCT/KR2022/011139**

(87) International publication number:
**WO 2023/008933 (02.02.2023 Gazette 2023/05)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **29.07.2021 KR 20210100125**

(71) Applicant: **Toolgen Incorporated
Seoul 07789 (KR)**

(72) Inventors:
• **SHIN, Eun Ji
Seoul 07789 (KR)**
• **LEE, Kang In
Seoul 07789 (KR)**
• **CHOI, Yu Ri
Seoul 07789 (KR)**
• **SHIN, Hye Jung
Seoul 07789 (KR)**
• **LEE, Jae Young
Seoul 07789 (KR)**
• **YU, Hwan Yeul
Seoul 07789 (KR)**

(74) Representative: **Modiano, Gabriella Diana et al
Modiano & Partners (DE)
Steinsdorfstrasse, 14
80538 München (DE)**

Remarks:
The complete document including Reference
Table(s) and the Sequence Listing(s) can be
downloaded from the EPO website

(54) **HEMOCOMPATIBLE MESENCHYMAL STEM CELLS, PREPARATION METHOD THEREFOR AND USE THEREOF**

(57)    The present invention presents mesenchymal stem cells, a preparation method therefor, and a cell therapeutic agent using same, the cells having hemocompatibility since thrombosis thereof is suppressed after the expression or activation level of blood coagulation initiating factor CD142 is reduced or inhibited. When intravascularly administered, the mesenchymal stem cells have improved hemocompatibility since thrombosis thereof, which can be caused by the administered mesenchymal stem cells, is suppressed.

EP 4 379 043 A1

[Fig.4]

**Description**

[Technical field]

The present disclosure provides hemocompatible mesenchymal stem cells that may be administered intravascularly due to the inhibition of the thrombogenic response because of the reduction or the inhibition of the expression or activity level of a blood coagulation initiating factor CD 142, a preparation method therefor, and a use thereof.

[B ackground Art]

Mesenchymal stem cells have self-proliferation and multi-potency, and because there are various types of progenitor cells, it is easy to secure stem cell lines, and they are multipotent stem cells and has the advantage of being much more genetically stable than pluripotent stem cells such as embryonic stem cells. In addition, due to its anti-inflammatory and immunomodulatory properties, it has been developed as a cell therapeutic agent for cartilage regeneration, myocardial infarction treatment, and graft versus host disease treatment.

Mesenchymal stem cells used as the cell therapeutic agent are administered intravenously or directly to the affected local. When mesenchymal stem cells are administered to an animal through intravascular injection, most of them accumulate in the lungs, but some may migrate to various organs such as an inflammation area, lymphocyte, liver, bones, and brain. The therapeutic mechanism of action of mesenchymal stem cells is known to be the secretion of paracrine factors, and immune-modulation is known to be the main therapeutic mechanism. Therefore, intravenously administered mesenchymal stem cells may cause immune-modulation in various organs of the body, so intravenous administration may be a preferred administration route.

However, unlike bone marrow (BM)-derived mesenchymal stem cells that are administered intravenously, fat cell-derived, umbilical cord blood/umbilical cord-derived, and perinatal tissue-derived mesenchymal stem cells are known to overexpress tissue factor (CD142, TF, factor III, and blood coagulation factor III, hereinafter CD142) and could cause blood coagulation, so it could not be administered intravenously due to the risk of causing venous thromboembolism (VTE), which is thrombosis and embolism in the veins. Bone marrow-derived mesenchymal stem cells also had limitations in dose when administered intravenously. In an in vitro/in vivo hemocompatibility experiment targeting human-derived mesenchymal stem cells, expression of CD142 was high in perinatal tissue (PT)-derived, umbilical cord blood/umbilical cord-derived mesenchymal stem cells (umbilical cord, UC) and fat tissue-derived mesenchymal stem cells (adipose tissue, AT) and there was a strong tendency for thrombus formation. Moreover, CD142-expressing perinatal tissue (PT)-derived, and umbilical cord blood/umbilical cord (UC)-derived mesenchymal stem cells, and adipose tissue-derived mesenchymal stem cells (adipose tissue (AT)) not only cause an Instant blood mediated inflammatory reaction (IBIR) to generate thrombus, but also tend to inhibit the engraftment of mesenchymal stem cells and increase the immune response, when administered intravenously (Guido Moll et al., Intravascular Mesenchymal Stromal/Stem Cell Therapy Product Diversification: Time for New Clinical Guidelines, Trends in Molecular Medicine, VOLUME 25, ISSUE 2, P149-163, FEBRUARY 01, 2019).

Animals have a blood coagulation system, which is a complex biological mechanism that prevents bleeding to minimize blood loss due to tissue damage. The blood coagulation system is a continuous amplification response process called a cascade response in response to an initial stimulus, and is composed of an intrinsic pathway directly triggered by the stimulus and an independent extrinsic pathway. Many types of blood coagulation factors are involved here.

Among them, CD142 is a cell membrane glycoprotein that acts in the first step of the extrinsic pathway coagulation process and plays an important role in the in vivo coagulation process. After binding to coagulation factors VII or VIIa, CD142 generates thrombin during the coagulation process by activating coagulation factor X, which is involved in activating factor IX and prothrombin into thrombin, and thrombin activates fibrinogen into fibrin, forming thrombus. In addition, CD142 activates immune cells, causing the activation of neutrophils, platelets, and monocytes, triggering the secretion of various cytokines, and activating the inherent immune system.

[Related Art Documents]

(Patent Document 1) US 2020-0016211

(Patent Document 2) JP 2016-140346

[Disclosure]

[Technical Problem]

The present disclosure is for the purpose of providing hemocompatible mesenchymal stem cells that inhibit a thrombogenic response that may be caused by the administered mesenchymal stem cells when administered intravascularly, a method of preparing the same, and a cell therapeutic agent using the same.

[Technical Solution]

In order to achieve the above object, the present disclosure provides hemocompatible mesenchymal stem cells that may be administered intravascularly by inhibiting the thrombogenic response by reducing or suppressing the expression or activity level of CD142, which is a blood coagulation initiating factor.

The present disclosure also provides an artificially engineered mesenchymal stem cell including an artificially engineered F3 gene, in which the artificially engineered F3 gene is different from the F3 gene sequence of a wild-type mesenchymal stem cell, the artificially engineered F3 gene includes one or more indels in the nucleic acid sequence, and an expression level of CD142 on the surface of the artificially engineered mesenchymal stem cell is characterized as reduced compared to wild-type mesenchymal stem cells.

In addition, the present disclosure provides a composition for preparing hemocompatible mesenchymal stem cells, including: a guide nucleic acid including a guide sequence capable of targeting a target sequence of the F3 gene of a mesenchymal stem cell, or a nucleic acid encoding the same; and an editor protein or a nucleic acid encoding the same.

The present disclosure also provides a method of preparing hemocompatible mesenchymal stem cells, including: (1) introducing a composition for preparing hemocompatible mesenchymal stem cells into isolated mesenchymal stem cells; and (2) editing the F3 gene to reduce or suppress the expression or activity of CD142 by generating an indel in the target sequence of the F3 gene located in the genome of the mesenchymal stem cell.

In addition, the present disclosure provides a cell therapeutic agent for vascular administration including, as an active ingredient, mesenchymal stem cells artificially engineered to have the above-described hemocompatibility.

In addition, the present disclosure provides a pharmaceutical composition including, as an active ingredient, mesenchymal stem cells artificially engineered to have the above-described hemocompatibility.

The present disclosure also provides a cell therapy method using stem cells, the method including intravascularly administering a therapeutically effective amount of the artificially engineered mesenchymal stem cell to a mammal having a disease or condition.

The present disclosure also provides the use of the artificially engineered mesenchymal stem cell for use in the manufacture of a medicament for inhibiting thrombus formation upon intravascular administration in a mammal having a disease or condition.

[Advantageous Effects]

The artificially engineered mesenchymal stem cells of the present disclosure can inhibit the blood coagulation mechanism by reducing or suppressing the expression or activity of CD142 to artificially modify the F3 gene encoding CD142 which is a blood coagulation initiating factor, using genome editing technology. Therefore, perinatal tissue (PT)-derived, and umbilical cord blood/umbilical cord (UC)-derived mesenchymal stem cells, and adipose tissue-derived mesenchymal stem cells (adipose tissue (AT)) can be administered intravenously, which were difficult to administer intravenously due to concerns of overexpression of CD142 on the surface of stem cells and causing venous thromboembolism (VTE), which causes thrombosis and embolism in the vein. In addition, the administration dose of bone marrow-derived mesenchymal stem cells, which were used in the existing intravenous administration route but had limitations in administration dose, can be increased. In addition, it may improve the engraftment of mesenchymal stem cells and reduce the inflammatory response, thereby increasing the efficacy of mesenchymal stem cells.

[Brief Description of Drawings]

FIG. 1 is a graph showing the results of measuring the indel efficiency for each guide RNA targeting the target sequence of SEQ ID NOs: 1 to 43 of F3 gene using a targeted deep sequencing method.

FIG. 2 is a graph showing the indel frequency in bone marrow-derived mesenchymal stem cells (F3 KO BM-MSC) in which the F3 gene was knocked out using the screened F3 target sgRNA.

FIG. 3 is a graph showing the indel frequency in umbilical cord blood-derived mesenchymal stem cells (F3 KO UC-MSC) in which the F3 gene was knocked out using the screened F3 target sgRNA (FIG. 3(a)), and the level of CD142 expression on the surface of umbilical cord blood-derived mesenchymal stem cells using FACS analysis (F3 KO UC-MSC) in which the F3 gene was knocked out (FIG. 3(b)).

FIG. 4 is a graph showing the level of CD142 expression on the surface of umbilical cord blood-derived mesenchymal stem cells (F3 KO UC-MSC) in which the F3 gene has been knocked out through FACS analysis using each guide RNA targeting the target sequence of SEQ ID NOs. 1 to 43 of the F3 gene.

FIG. 5 shows an experimental schedule according to passage of umbilical cord blood-derived mesenchymal stem cells (US-MSC) in an experimental example according to an embodiment of the present disclosure.

FIG. 6 is a graph showing the results of measuring indel efficiency through a targeted deep sequencing method (FIG. 6(a)); and F3 mRNA expression level (FIG. 6(b)) for wild-type umbilical cord blood-derived stem cells (WT), umbilical cord blood-derived stem cells with electroporation alone and no gene engineering (Mock), and umbilical cord blood-derived stem cells with knockout of the target sequence of SEQ ID NO: 31 (sgCD142#33).

FIG. 7 shows the results of microscopic observation of the morphology of mesenchymal stem cells after transfection.

FIG. 8 is a graph showing population doubling level (PDL) (FIG. 8(a)) and population doubling time (PDT) (FIG. 8(b)) of wild-type umbilical cord blood-derived stem cells (WT), umbilical cord blood-derived stem cells with electroporation alone and no gene engineering (Mock), and umbilical cord blood-derived stem cells with knockout of the target sequence of SEQ ID NO: 31(sgCD142#33).

FIG. 9 is a graph showing the percentage of cells showing CD142 expression (FIG. 9(a)) and CD142 median fluorescence intensity (MFI) (FIG. 9(b)) of wild-type umbilical cord blood-derived stem cells (WT), umbilical cord blood-derived stem cells with electroporation alone and no gene engineering (Mock), and umbilical cord blood-derived stem cells with knockout of the target sequence of SEQ ID NO: 31 (sgCD142#33).

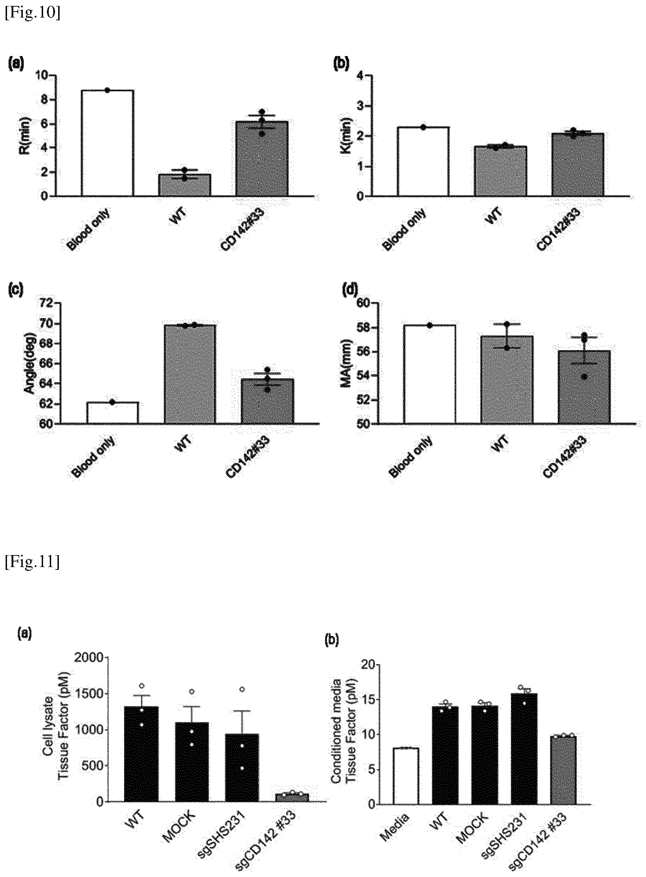

FIG. 10 is a graph showing coagulation response time (R-time: time from the start of analysis until the thrombus amplitude reaches 2 mm) (FIG. 10(a)), thrombus dynamics (K-time: time from when the thrombus amplitude reaches 2 mm until the amplitude reaches 20 mm) (FIG. 10(b)), thrombus formation intensity (alpha angle: the angle formed by the tangent at the midpoint of R and K times) (FIG. 10(c)), and thrombus maximum amplitude (MA: absolute thrombus strength) (FIG. 10(d)) of wild-type umbilical cord blood-derived mesenchymal stem cells (WT), umbilical cord blood-derived mesenchymal stem cells with electroporation alone and no gene engineering (Mock), and umbilical cord blood-derived stem cells with knockout of the target sequence of SEQ ID NO: 31 (sgCD142#33), when measuring using TEG 6s equipment.

FIG. 11 is a graph showing tissue factor in cell lysate (FIG. 11(a)) and tissue factor in conditioned media (FIG. 11(b)) of wild-type umbilical cord blood-derived stem cells (WT), umbilical cord blood-derived stem cells with electroporation alone and no gene engineering (Mock), and umbilical cord blood-derived stem cells with knockout of the target sequence of SEQ ID NO: 31 (sgCD142#33).

FIG. 12 is a table showing a list of genes with a p-value of less than 0.05 and a normalized data (log 2) value of 4 or more as a result of RNA-seq (QuantSeq) analysis.

FIG. 13 is a table showing the results of secretome analysis through antibody microarray.

[Best Mode for Implementation of the Invention]

Hereinafter, the present disclosure will be described in more detail.

As used herein, the term "about" refers to quantity, level, value, number, frequency, percentage, dimension, size, amount, weight, or length that vary by 30, 25, 20, 25, 10, 9, 8, 7, 6, 5, 4, 3, 2 or 1 degrees with respect to a reference quantity, level, value, number, frequency, percentage, dimension, size, amount, weight, or length.

As used herein, the term "artificially engineered" is a term used to distinguish substances, molecules and the like that have a composition that is already present in the natural world, and means that artificial modifications have been made to the substances, molecules and the like. For example, the expression "artificially engineered gene" refers to a gene in which artificial modifications have been made to the composition of genes that are present in the natural world. In addition, the above term includes all meanings that may be recognized by a person skilled in the art, and may be appropriately interpreted depending on the context.

As used herein, the term "wild-type" refers to that a gene including a naturally occurring base sequence and a protein expressed from the gene have normal functional characteristics. Wild-type genes have a form in which no natural or artificial mutations have occurred and are most frequently observed in the population. When the term "wild-type" is used herein in contrast to artificially engineered genes and/or artificially engineered cells, this may be interpreted to mean a gene including an artificially engineered gene and/or a homologous "non-artificially engineered" naturally occurring base sequence corresponding to the artificially engineered cell, and a cell having the same. In addition, the above term includes all meanings that may be recognized by a person skilled in the art, and may be appropriately interpreted depending on the context.

As used herein, the expression "knock-out" or "knocked out gene" means that a mutation or artificial modification occurs in a wild-type gene, and as a result, means that the protein expressed by the wild-type gene is not produced through transcription and/or translation processes. For example, cells including knocked-out gene A may be unable to express the mRNA and/or protein expressed by wild-type gene A. A cell including a knocked-out gene A may be one in which only one gene A present in the cell is knocked out, or two or more genes A may be knocked out. In addition, the above term includes all meanings that may be recognized by a person skilled in the art, and may be appropriately interpreted depending on the context.

As used herein, the expression "knock-down" or "knocked down gene" means that a mutation or artificial modification occurs in a wild-type gene, resulting in the expression of a material in a lower amount than the wild-type gene. For example, cells including knocked down gene A may express less mRNA than that expressed by the wild-type gene A. As another example, cells including knocked down gene A may express a smaller amount of protein than the protein expressed by the wild-type gene A. A cell in which gene A has been knocked down may have only one gene A present in the cell knocked down, or have two or more genes knocked down. In addition, the above term includes all meanings that may be recognized by a person skilled in the art, and may be appropriately interpreted depending on the context.

As used herein, "expression reduction" means showing a lower level of expression than the expression level of mRNA and/or protein measured in the wild-type. The reduction may be a reduction of about 5% or more, about 10% or more, about 15% or more, about 20% or more, about 30% or more, about 50% or more, about 60% or more, about 70% or more, or about 100% or more compared to cells without genetic modification or wild-type cells.

As used herein, the term "activity reduction" or "reduced activity" may mean a reduction in relative activity when measuring protein or enzyme activity. Specifically, "activity reduction" or "reduced activity" means lower levels of protein, or enzyme activity compared to given parental or wild-type cells.

As used herein, the term "hemocompatibility" means not causing hemolysis, platelet adhesion, platelet activation, fibrin formation through complement activation, thrombogenic response, or embolism upon contact with blood.

As used herein, the term "stem cell" refers to a broad concept that collectively refers to undifferentiated cells with sternness, that is, the ability to differentiate into various types of body tissue cells. At this time, the stem cells may be induced pluripotent stem cells, embryonic stem cells, and adult stem cells. In addition, the cells may be of human origin, but are not limited thereto.

As used herein, the expression "mesenchymal stem cell" is undifferentiated stem cells isolated from human or mammalian tissues, and may be derived from various tissues. In particular, the mesenchymal stem cells may be umbilical cord-derived mesenchymal stem cells, umbilical cord blood-derived mesenchymal stem cells, bone marrow-derived mesenchymal stem cells, fat-derived mesenchymal stem cells, muscle-derived mesenchymal stem cells, nerve-derived mesenchymal stem cells, skin-derived mesenchymal stem cells, amniotic membrane-derived mesenchymal stem cells, amniotic fluid-derived mesenchymal stem cells, perinatal tissue-derived mesenchymal stem cells, or placenta-derived mesenchymal stem cells, and technologies for isolating stem cells from each tissue are already known in the art.

The present disclosure provides mesenchymal stem cells in which the expression or activity of CD142 (tissue factor, TF, factor III, tissue factor, blood coagulation factor III) is reduced or suppressed.

Mesenchymal stem cells with reduced or suppressed expression or activity of CD142 are cell membrane glycoproteins that act in the first step of an exogenous coagulation process, and are mesenchymal stem cells in which the blood coagulation mechanism is inhibited by reducing or suppressing the expression or activity of CD142, which plays an important role in the blood coagulation process in vivo, and when utilized as a cell therapeutic agent and administered through the intravenous route, mesenchymal stem cells with reduced or suppressed expression or activity of CD 142 may exhibit hemocompatibility showing improved hemocompatibility over wild-type mesenchymal stem cells, mesenchymal stem cells artificially engineered by techniques in the related art, or CD142 low-expressing mesenchymal stem cells selected with antibodies, cell sorting devices, or magnetic beads.

Specifically, the present disclosure provides hemocompatible artificially engineered mesenchymal stem cells characterized by reduced blood coagulation response when administered intravenously by artificially engineering the F3 gene of mesenchymal stem cells to reduce or inhibit the expression or activity of F3 mRNA and/or CD142

The present disclosure provides artificially engineered mesenchymal stem cells including an artificially modified nucleic acid sequence of the F3 gene.

In the present disclosure, "artificial modification" or "artificial engineering" of a gene nucleic acid sequence may be achieved through modification of the nucleic acid sequence constituting the gene or chemical modification of a single base. This may be due to mutation, substitution, or deletion of part or all of the genes, or the insertion of one or more bases into the gene, and may be achieved using gene editing technology such as the CRISPR-enzyme system. As an example, artificial modification of the gene nucleic acid sequence may be achieved by non-homologous end joining (NHEJ) or homology directed repair (HDR) mechanisms.

As an example, the artificially engineered mesenchymal stem cells may have the F3 gene knocked out.

As used herein, "non-homologous end joining (NHEJ)" is a method of restoring or repairing a double-strand break in DNA by joining both ends of a cut double-strand or single strand together, and when two compatible ends, usually formed by a break in the double strand (e.g., cleavage), repeat frequent contacts such that the two ends become fully joined, broken double strands are repaired.

In the process of repairing damaged genes or nucleic acids using NHEJ, some "insertion and/or deletion" (or "indels", InDels) of nucleic acid sequences may occur at the NHEJ repair site, and the gene in which an indel occurred does not have the same sequence as the wild-type gene. This insertion and/or deletion changes the reading frame of the gene, produce frame shifted transcript mRNA, and eventually loses its original function by undergoing nonsense-mediated decay or failing to synthesize normal proteins. In addition, mutations may occur that maintain the reading frame but insert or delete significant amounts of sequence, destroying the functionality of the protein. As another example, when an indel occurs in a transcriptional regulatory region such as the promoter region or enhancer region of a gene, the mRNA may not be transcribed or the transcription amount may be reduced, and thus the protein may not be expressed or the expression amount may be reduced. Alternatively, the mutagenesis mechanism of NHEJ may be utilized to delete only some sequence motifs when the generation of a specific final sequence is not required. For example, when two or more guide RNAs targeting the 5' and 3' intron regions of a specific exon are used to cause a double-strand break in each intron, and only part of the exon of the gene is deleted by NHEJ, other portions can be expressed normally and the main functionality of the protein may be maintained.

Using this NHEJ, the gene of interest may be specifically knocked out or knocked down using genome editing technology.

For example, CRISPR enzymes such as Cas9 or Cpf1, a type of genetic scissors, are used to cut double strands or two single strands of the target gene or target nucleic acid, indels re generated by NHEJ on a double strand or two single strands of a broken target gene or a broken target nucleic acid, and through this, specific knock-out or knock-down of the target gene or nucleic acid may be induced.

As an example, the artificially engineered mesenchymal stem cells may have the F3 gene knocked down.

In one embodiment of the present disclosure, the F3 gene of the artificially engineered mesenchymal stem cell may include one or more indels in the nucleic acid sequence.

In one embodiment of the present disclosure, the sequence of the F3 gene of the artificially engineered mesenchymal stem cell may not include one or more sequences selected from the group consisting of SEQ ID NOs: 1 to 43.

In one embodiment of the present disclosure, the artificially engineered mesenchymal stem cells may not express F3 mRNA.

In one embodiment of the present disclosure, the mRNA transcribed from the artificially engineered F3 gene of the artificially engineered mesenchymal stem cell may have lower expression levels of that mRNA compared to the level of mRNA transcribed from the F3 gene of wild-type mesenchymal stem cells.

In one embodiment of the present disclosure, the artificially engineered mesenchymal stem cells may have different F3 mRNA sequences compared to wild-type stem cells.

In one embodiment of the present disclosure, the artificially engineered mesenchymal stem cells may have reduced expression or activity of CD142 on the cell surface compared to wild-type stem cells. Through this, the function of CD142 in the stem cells of the present disclosure may be reduced or lost.

That is, in the artificially engineered mesenchymal stem cells, the expression or activity of CD142 may be reduced by about 20% or more, about 30% or more, about 40% or more, about 50% or more, about 55% or more, about 60% or more, about 70% or more, about 75% or more, about 80% or more, about 85% or more, about 90% or more, about 95% or more, or about 100% compared to the expression or activity of wild-type mesenchymal stem cells.

The present disclosure provides a method of preparing hemocompatible artificially engineered mesenchymal stem cells, including reducing the expression or activity of CD142 of the mesenchymal stem cells.

The increase or decrease in the expression or activity of CD142 may be achieved by artificial modification of the F3 gene, for example, using gene editing technology.

As an example, the genome editing technology may utilize TALEN (transcription activator like effector nuclease) in which a transcription activator-like (TAL) effector (TALE) domain and a cutting domain are fused, a zinc-finger nuclease, or CRISPR-enzyme system derived from a clustered regularly interspaced short palindromic repeats (CRISPR), which is a microbial immune system, but is not limited thereto.

The entire contents disclosed in International Patent Publication No. WO2012/093833 or U.S. Patent Publication No. 2013-0217131 with respect to the above TALEN are incorporated in the present specification by reference. In relation to the above ZFN, Beerli et al. (2002) Nature Biotechnol. 20:135-141; Pabo et al. (2001) Ann. Rev. Biochem. 70:313-340; Isalan et al, (2001) Nature Biotechnol. 19: 656-660; Segal et al. (2001) Curr. Opin. Biotechnol. 12:632-637; Choo et al. (2000) Curr. Opin. Struct. Biol.10:411-416; and U.S. Patent Nos. 7,888,121, 8,409,861, 6,479,626, 6,903,185, and 7,153,949 may be included as reference material in the present specification.

The "CRISPR-enzyme system" consists of a guide nucleic acid and/or an editor protein.

"Guide nucleic acid" refers to a nucleic acid capable of recognizing a target nucleic acid, target gene, or target chromosome and interacting with an editor protein. At this time, the guide nucleic acid may form a complementary bond with some nucleotides in the target nucleic acid, target gene, or target chromosome.

The guide nucleic acid may be in the form of a target DNA-specific guide RNA, DNA encoding the guide RNA, or a DNA/RNA mixture.

The guide nucleic acid may be a guide RNA. As an example, the "guide RNA" may be transcribed in vitro, particularly from an oligonucleotide double strand, or a plasmid template. As another example, the guide RNA may be encoded in the form of a vector, and may be transferred into a cell in an ex vivo or in vivo environment and transcribed from the vector, but is not limited thereto.

The design and composition of the guide RNA are known to those skilled in the art and are described in detail in Korean Registration Patents 10-1656236, 10-1656237, 10-1706085, 10-2052286, and 10-2182847, and the entirety of the above Registration Patents is incorporated herein as reference material for the present disclosure.

The guide nucleic acid may include a scaffold sequence portion and a guide sequence portion. The scaffold sequence portion is a portion that interacts with the Cas protein and allows the Cas protein and a guide nucleic acid to bind to form a complex (ribonucleoprotein, RNP). Generally, the scaffold sequence portion includes tracrRNA and some sequence portions of crRNA, and the scaffold sequence is determined depending on which Cas protein is used.

The guide sequence portion is a nucleotide sequence portion that may bind complementary to some sequences of any one of the double strands of a target gene or nucleic acid, and a nucleotide sequence portion that may be artificially modified, and is determined by the target nucleotide sequence of interest. At this time, the guide sequence may be a nucleotide sequence having at least 50%, 55%, 60%, 65%, 70%, 75%, 80%, 85%, 90%, or 95% or more complementarity or complete complementarity with the guide nucleic acid binding sequence of the target gene or target nucleic acid. The guide sequence may be a sequence included in the guide domain of the guide nucleic acid.

The guide sequence portion may be included in crRNA. As an example, the guide nucleic acid may be a dual RNA including two RNAs, namely, crRNA (CRISPR RNA) and tracrRNA (trans-activating crRNA) as components.

As another example, the guide nucleic acid may be a single-chain guide RNA (sgRNA) in which the main portions of crRNA and tracrRNA are linked.

The target sequence may be a nucleotide sequence of a certain length present in the target gene or target nucleic acid, and specifically, some nucleotide sequences within the target region classified by a regulatory region of the target gene, coding region (or CDS, coding sequence), or a non-coding region (or untranslated region (UTR)), or one or more some nucleotide sequences selected from a combination of the target regions. The target sequence may be a target of a guide nucleic acid-editor protein complex (RNP).

In one embodiment of the present disclosure, the target sequence may be a sequence included in a first region, second region, third region, fourth region, or sixth region of the exon of the wild-type F3 gene.

In one embodiment of the present disclosure, the target sequence is one or more sequences selected from SEQ ID NOs: 1 to 43.

The target sequence is a nucleotide sequence adjacent to a protospacer-adjacent motif (PAM) sequence recognized by the editor protein, and may include all or part of the PAM, but is not limited thereto.

The term "target sequence" may be used to mean both types of nucleotide sequence information. For example, in the case of a target gene, the target sequence may mean sequence information of the transcribed strand of the target gene DNA, or may mean nucleotide sequence information of the non-transcribed strand.

The target sequence includes a guide nucleic acid binding sequence or a guide nucleic acid-nonbinding sequence. The guide nucleic acid binding sequence is a nucleotide sequence that has partial or complete complementarity with the guide sequence included in the guide domain of the guide nucleic acid, and may bind complementary to the guide sequence included in the guide domain of the guide nucleic acid. The target sequence and guide nucleic acid binding sequence are nucleotide sequences that may vary depending on the target gene or nucleic acid, that is, depending on the subject to be genetically engineered or corrected, and the guide nucleic acid may be designed in various ways depending on the target gene or target nucleic acid.

The guide nucleic acid non-binding sequence is a nucleotide sequence that has partial or complete complementarity with the guide sequence included in the guide domain of the guide nucleic acid, and cannot bind complementary to the guide sequence included in the guide domain of the guide nucleic acid. In addition, the guide nucleic acid non-binding sequence is a nucleotide sequence that is complementary to the guide nucleic acid binding sequence and may bind complementary to the guide nucleic acid binding sequence. The guide nucleic acid binding sequence is some nucleotide sequences of the target sequence, and may be a nucleotide sequence having two different sequence orders of the target sequence, that is, one nucleotide sequence of two nucleotide sequences capable of complementary binding to each other. At this time, the guide nucleic acid non-binding sequence may be the remaining nucleotide sequence of the target sequence excluding the guide nucleic acid binding sequence.

The guide nucleic acid binding sequence may be a target sequence, that is, one nucleotide sequence selected from a nucleotide sequence identical to the transcribed strand and a nucleotide sequence identical to the non-transcribed strand. At this time, the guide nucleic acid non-binding sequence may be the guide nucleic acid binding sequence among the target sequences, that is, the remaining nucleotide sequence excluding one nucleotide sequence selected from the nucleotide sequence identical to the transcribed strand and the nucleotide sequence identical to the non-transcribed strand.

The guide nucleic acid binding sequence may be the same length as the target sequence. The guide nucleic acid non-binding sequence may be the same length as the target sequence or guide nucleic acid binding sequence. The guide nucleic acid binding sequence may be 5 to 50 nucleotide sequences.

In one embodiment, the guide nucleic acid binding sequence is 16 nucleotide sequences, 17 nucleotide sequences, 18 nucleotide sequences, 19 nucleotide sequences, 20 nucleotide sequences, 21 nucleotide sequences, 22 nucleotide sequences, 23 nucleotide sequences, 24 nucleotide sequences, or 25 nucleotide sequences. The guide nucleic acid non-binding sequence may be 5 to 50 nucleotide sequences.

In one embodiment, the guide nucleic acid non-binding sequence is 16 nucleotide sequences, 17 nucleotide sequences, 18 nucleotide sequences, 19 nucleotide sequences, 20 nucleotide sequences, 21 nucleotide sequences, 22 nucleotide sequences, 23 nucleotide sequences, 24 nucleotide sequences, or 25 nucleotide sequences.

The guide nucleic acid binding sequence may form a partial or complete complementary bond with the guide sequence included in the guide domain of the guide nucleic acid, and the length of the guide nucleic acid binding sequence may be the same as the length of the guide sequence.

The guide nucleic acid binding sequence may be a nucleotide sequence complementary to the guide sequence included in the guide domain of the guide nucleic acid, for example, a nucleotide sequence that is at least 70%, 75%, 80%, 85%, 90%, or 95% complementary or completely complementary.

As an example, the guide nucleic acid binding sequence may have or include a 1 to 8 nucleotide sequences that is not complementary to the guide sequence included in the guide domain of the guide nucleic acid.

The guide nucleic acid non-binding sequence may have partial or complete homology to the guide sequence included in the guide domain of the guide nucleic acid, and the length of the guide nucleic acid non-binding sequence may be the same as the length of the guide sequence. As an example, the guide sequence may be designed based on a sequence having homology to the guide nucleic acid non-binding sequence.

The guide nucleic acid non-binding sequence may be a nucleotide sequence having homology to the guide sequence included in the guide domain of the guide nucleic acid, for example, a nucleotide sequence having at least 70%, 75%, 80%, 85%, 90%, or 95% homology or completely homology.

As an example, the guide nucleic acid non-binding sequence may have or include a 1 to 8 nucleotide sequences that is not homologous to the guide sequence included in the guide domain of the guide nucleic acid. The guide nucleic acid non-binding sequence may bind complementary to the guide nucleic acid binding sequence, and the guide nucleic acid non-binding sequence may be the same as the length of the guide nucleic acid binding sequence.

The guide nucleic acid non-binding sequence may be a nucleotide sequence complementary to the guide nucleic acid binding sequence, for example, a nucleotide sequence that is at least 90% or 95% complementary or completely complementary.

As an example, the guide nucleic acid non-binding sequence may have or include 1 to 2 nucleotide sequences that are not complementary to the guide nucleic acid binding sequence. In addition, the guide nucleic acid binding sequence may be a nucleotide sequence located close to a complementary sequence complementary to a nucleotide sequence (PAM sequence) that may be recognized by the editor protein.

As an example, the guide nucleic acid binding sequence may be a contiguous 5 to 50 nucleotide sequences located adjacent to the 5' end or/and 3' end of a sequence complementary to a nucleotide sequence (PAM sequence) that may be recognized by the editor protein.

In addition, the guide nucleic acid non-binding sequence may be a nucleotide sequence located close to a nucleotide sequence (PAM sequence) that may be recognized by the editor protein.

As an example, the guide nucleic acid non-binding sequence may be a contiguous 5 to 50 nucleotide sequences located adjacent to the 5' end or/and 3' end of a nucleotide sequence (PAM sequence) that may be recognized by the editor protein.

"Editor protein" means a peptide, polypeptide or protein that either bind directly to nucleic acids, or do not bind directly, but may interact a nucleic acid. The editor protein is conceptually referred to as "artificially engineered nuclease" or RNA-guided endonuclease RGEN).

In one embodiment, the editor protein may be a CRISPR enzyme. "CRISPR enzyme" is the main protein component of the CRISPR-enzyme system, also called "Cas protein (CRISPR associated protein)", and refers to a nuclease that may recognize a target sequence and cleave DNA by mixing or forming a complex with a guide RNA.

CRISPR enzymes are known to those skilled in the art, see Korean Registration Patents Nos. 10-1656236, 10-1656237, 10-1706085, 10-2052286, and 10-2182847. The CRISPR enzyme is used herein as a concept that includes all variants that may act as an endonuclease or nickase activated in cooperation with a guide RNA, in addition to the native protein. In the case of activated endonuclease or nickase, target DNA may be cut and this may be used to perform genome editing. In addition, in the case of an inactivated variant, the genome editing may be used to regulate transcription or isolate the desired DNA.

The CRISPR enzyme is a nucleic acid or polypeptide (or protein) having a sequence encoding the CRISPR enzyme, typically, Type II CRISPR enzyme or Type V CRISPR enzyme is widely used, and the Type II CRISPR enzyme is CRISPR associated protein 9 (Cas9) protein.

The Cas9 protein may be derived from various microorganisms such as *Streptococcus pyogenes, Streptococcus thermophilus, Streptococcus* sp., *Campylobacter jejuni, Staphylococcus aureus, Staphylococcus Auricularis,* and *Neisseria meningitidis.*

In order for the Cas9 protein to induce a double-stranded DNA break, the Cas9 protein recognizes a protospacer-adjacent motif (PAM) sequence, which is a nucleotide sequence of a certain length, and requires that a portion of the guide RNA (the guide sequence portion) bind complementary to the complementary strand (the guide nucleic acid binding sequence) of a single strand of DNA (the guide nucleic acid non-binding sequence) on which the target sequence is located.

This PAM sequence is a sequence determined depending on the type or origin of the Cas9 protein, for example, a *Streptococcus pyogenes*-derived Cas9 protein (SpCas9) may recognize the 5'-NGG-3' sequence (complementary sequence: 5'-CCN-3') in the target nucleic acid. At this time, N is one of adenosine (A), thymidine (T), cytidine (C), and guanosine (G). In addition, SpCas9 can recognize the 5'-NAG-3' sequence (complementary sequence: 5'-CTN-3') in the target nucleic acid with low activity.

In addition, the Type V CRISPR enzyme includes Cpf1, and Cpf1 may be *Streptococcus, Campylobacter, Nitratifractor, Staphylococcus, Parvibaculum, Roseburia, Neisseria, Gluconacetobacter, Azospirillum, Sphaerochaeta,Lactobacillus, Eubacterium, Corynebacter, Carnobacterium, Rhodobacter, Listeria, Paludibacter, Clostridium, Lachnospiraceae, Clostridiaridium, Leptotrichia, Francisella, Legionella, Alicyclobacillus, Methanomethyophilus, Porphyromonas, Prevotella, Bacteroidetes, Helcococcus, Letospira, Desulfovibrio, Desulfonatronum, Opitutaceae, uberibacillus, Bacillus, Brevibacilus, Methylobacterium,* or *Acidaminococcus*-derived Cpf1.

CRISPR enzymes such as the Cas9 or Cpf1 protein may be isolated from microorganisms present in nature or produced unnaturally through recombinant or synthetic methods. The Cas protein may also be in a form that is easy to introduce into cells. For example, the Cas protein may be linked to a cell-penetrating peptide or protein transduction domain. The protein transduction domain may be poly-arginine or HIV-derived TAT protein, but is not limited thereto. Since various types of cell-penetrating peptides or protein transduction domains are known in the art in addition to the examples described above, those skilled in the art are not limited to the above examples and may apply various examples to the present specification. In addition, the Cas protein may be fused with a functional domain such as a nuclear localization sequence or signal (NLS). In addition, the Cas9 protein may be encoded in the form of a vector and expressed within cells.

The present disclosure provides a composition for preparing hemocompatible mesenchymal stem cells, including: a guide nucleic acid including a guide sequence capable of targeting a target sequence of the F3 gene of a mesenchymal stem cell, or a nucleic acid encoding the same; and an editor protein or a nucleic acid encoding the same.

In addition, the composition may optionally further include a donor including a specific nucleotide sequence to be inserted or a nucleic acid encoding the same.

The donor refers to exogenous nucleotide sequences that may express a specific peptide or protein, and may be inserted into genomic DNA through homology directed repair (HDR).

The donor may be a double-stranded nucleic acid or a single-stranded nucleic acid. The donor may be linear or circular.

The donor may be in the form of a viral vector or a non-viral vector (e.g., a plasmid).

The virus may be a DNA virus or an RNA virus. At this time, the DNA virus may be a double-stranded DNA (dsDNA) virus or a single-stranded DNA (ssDNA) virus. At this time, the RNA virus may be a single-stranded RNA (ssRNA) virus.

The viral vector may be one or more viral vectors selected from the group consisting of retrovirus, lentivirus, adenovirus, adeno-associated virus (AAV), vaccinia virus, poxvirus, and herpes simplex virus (HSV).

The target sequence may be a target of a guide nucleic acid-editor protein complex, and the target sequence may include a protospacer-adjacent motif (PAM) sequence recognized by the editor protein, but is not limited thereto.

The guide nucleic acid may include a guide domain capable of targeting the target sequence of the F3 gene.

In one embodiment of the present disclosure, the target sequence of the composition may be one or more sequences selected from SEQ ID NOs: 1 to 42.

In the present specification, the guide nucleic acid, editor protein or guide nucleic acid-editor protein complex (ribonucleoprotein, RNP) and/or donor may be delivered or introduced into the subject in various forms.

In this case, the "subject" refers to an organism into which the guide nucleic acid, editor protein, or guide nucleic acid-editor protein complex is introduced; an organism in which a guide nucleic acid, an editor protein, or a guide nucleic acid-editor protein complex operates; or a specimen or sample obtained from the organism.

The subject may be an organism including a target gene, a target nucleic acid, or a target chromosome of the guide nucleic acid-editor protein complex.

The organism may be an animal, animal tissue, or animal cell. At this time, the tissue may be the eye, skin, liver, kidney, heart, lung, brain, muscle, or blood.

The cells may be stem cells, liver cells, cardiac muscle cells, endothelial cells, or pancreatic cells.

The specimen or sample may be acquired from an organism including target gene, target nucleic acid, or target chromosome, such as saliva, blood, liver tissues, brain tissues, liver cells, nerve cells, phagocytes, macrophages, T cells, B cells, astrocytes, cancer cells, or stem cells.

The guide nucleic acid, editor protein, or guide nucleic acid-editor protein complex may be delivered or introduced into the subject in the form of DNA, RNA, or a mixture thereof.

At this time, DNA, RNA, or a mixture thereof encoding the guide nucleic acid and/or the editor protein may be delivered or introduced into the subject by methods known in the art.

Alternatively, the form of DNA, RNA, or a mixture thereof encoding the guide nucleic acid and/or the editor protein may be delivered or introduced into the subject by vector, non-vector, or a combination thereof.

The vector may be a viral vector or a non-viral vector (e.g., a plasmid).

The virus may be a DNA virus or an RNA virus. At this time, the DNA virus may be a double-stranded DNA (dsDNA) virus or a single-stranded DNA (ssDNA) virus. At this time, the RNA virus may be a single-stranded RNA (ssRNA) virus.

The viral vector may be one or more selected from the group consisting of retrovirus, lentivirus, adenovirus, adeno-associated virus (AAV), vaccinia virus, poxvirus, and herpes simplex virus.

The non-vector may be naked DNA, DNA complex, or mRNA.

In one embodiment of the present disclosure, the nucleic acid encoding the guide nucleic acid and/or the editor protein may be delivered or introduced into the subject in the form of one or more vectors.

The vector may include a guide nucleic acid and/or a nucleic acid encoding an editor protein. As an example, the vector may simultaneously include a guide nucleic acid and a nucleic acid encoding an editor protein. As another example, the vector may include a nucleic acid encoding a guide nucleic acid. For example, the nucleic acid encoding the guide nucleic acid may be all included in one vector, or the nucleic acid encoding the guide nucleic acid may be divided and included in multiple vectors. As another example, the vector may include a nucleic acid encoding an editor protein. For example, in the case of the editor protein, the nucleic acid encoding the editor protein may be included in one vector, or the nucleic acid encoding the editor protein may be divided and included in multiple vectors.

The editor protein may be delivered or introduced into the subject in the form of peptide, polypeptide, or protein.

The editor protein may be delivered or introduced into the subject in the form of peptide, polypeptide, or protein by methods known in the art.

The guide nucleic acid and the editor protein may be delivered or introduced into the subject in the form of a nucleic acid-protein mixture.

The guide nucleic acid and the editor protein may be delivered or introduced into the subject in the form of a guide nucleic acid-editor protein complex. For example, the guide nucleic acid may be DNA, RNA, or a mixture thereof. The editor protein may be in the form of peptide, polypeptide, or protein. As an example, in the case of the guide nucleic acid and the editor protein, a guide nucleic acid in the form of RNA and an editor protein in the form of a protein may be delivered or introduced into the subject in the form of a guide nucleic acid-editor protein complex, that is, ribonucleoprotein (RNP).

In addition, the present disclosure provides a method of preparing hemocompatible mesenchymal stem cells, including: (1) introducing a composition for preparing hemocompatible mesenchymal stem cells into isolated mesenchymal stem cells; and (2) editing the F3 gene to reduce or suppress the expression or activity of CD142 by generating an indel in the target sequence of the F3 gene located in the genome of the mesenchymal stem cell.

At this time, the "introduction" may be performed by one or more means selected from electroporation, lipofection, microinjection, genetic blueprint, liposomes, positive liposomes, plasmids, viral vectors, nanoparticles, protein translocation domain (PTD) Methods, immunoliposomes, polycations or lipids: nucleic acid conjugates, naked DNA, artificial virions, and preparation-enhanced uptake methods of DNA, but is not limited thereto.

In one embodiment of the present disclosure, hemocompatible mesenchymal stem cells may be prepared by introducing a composition for preparing hemocompatible mesenchymal stem cells into isolated mesenchymal stem cells by electroporation.

In one embodiment of the present disclosure, a CRISPR/Cas9 complex including Streptococcus pyogenes-derived Cas9 protein and guide RNA that may target the target sequence of the F3 gene is contacted with the F3 gene located within the genome of the mesenchymal stem cell, thereby generating an indel within the target sequence.

In one embodiment of the present disclosure, the target sequence may be one or more sequences selected from the group consisting of SEQ ID NOs: 1 to 43.

The hemocompatible artificially engineered mesenchymal stem cells of the present disclosure may be used as a cell therapeutic agent or a pharmaceutical composition for vascular administration and may be used to treat various diseases. For example, the stem cells may be used for heart disease, gastroduodenal disease, Small and large intestine diseases, liver disease, biliary tract disease, pancreas disease, kidney disease, liver disease, lung disease, mediastinal disease, diaphragm disease, pleural disease, peritoneal disease, neurological disease, central nervous system (CNS) disorder, peripheral arterial disease, and peripheral venous disease. Specific diseases may include, for example, liver diseases such as autoimmune hepatitis, fulminant hepatitis, chronic hepatitis, viral hepatitis, alcoholic hepatitis, nonalcoholic fatty liver disease (NAFLD), and nonalcoholic steatohepatitis (NASH), nonalcoholic fatty liver (NAFL), liver fibrosis, cirrhosis, liver cancer, fatty liver, drug-induced allergic liver disease, hemosiderosis, hemochromatosis, Wilson's disease, primary biliary cirrhosis (PBC), primary sclerosing cholangitis (PSC), biliary atresia, liver abscess, chronic active hepatitis, and chronic persistent hepatitis; heart diseases such as myocardial infarction, heart failure, arrhythmia, palpitations, cardiomyopathy, ischemic cardiomyopathy, angina pectoris, congenital heart disease, valvular disease, myocarditis, familial hypertrophic cardiomyopathy, dilated cardiomyopathy, acute coronary syndrome, arteriosclerosis, and restenosis; gastroduodenal diseases such as acute gastritis, chronic gastritis, gastroduodenal ulcer, stomach cancer, and duodenal cancer; Small and large intestine diseases such as ischemic enteritis, inflammatory bowel disease, ulcerative colitis, Crohn's disease, simple ulcer, intestinal Behcet's disease, small intestine cancer, and colon cancer; biliary duct diseases such as acute cholecystitis, acute cholangitis, chronic cholecystitis, cholangiocarcinoma, and gallbladder cancer; pancreatic diseases such as acute pancreatitis, chronic pancreatitis, and pancreatic cancer; kidney diseases such as acute nephritis, chronic nephritis, acute renal failure, and chronic renal failure; lung diseases, such as pneumonia, emphysema, pulmonary fibrosis, interstitial pneumonia, idiopathic interstitial pneumonia, exfoliative interstitial pneumonia, acute interstitial pneumonia, non-specific interstitial pneumonia, drug-induced lung disease, eosinophilic lung disease, pulmonary hypertension, pulmonary tuberculosis, sequelae of pulmonary tuberculosis , acute respiratory distress syndrome, cystic fibrosis, chronic obstructive pulmonary disease, pulmonary embolism, pulmonary abscess, pneumoconiosis, aspiration pneumonia, pulmonary fibrosis, acute upper respiratory tract infection, chronic lower respiratory tract infection, pneumothorax, alveolar epithelial damage disease, lymphangioleiomyoma, lymphatic interstitial pneumonia, alveolar proteinosis, and pulmonary Langerhans cell granulomatosis; mediastinal diseases such as mediastinal tumor, mediastinal cystic disease, and mediastinitis; diaphragmatic disease such as diaphragmatic hernia; pleural diseases such as pleurisy, empyema, pleural tumor, cancerous pleurisy, and pleural mesothelioma; peritoneal diseases such as peritonitis and peritoneal tumor; neurological diseases such as cerebral palsy syndrome, including pediatric cerebral palsy, aseptic meningitis, Guillain-Barre syndrome, amyotrophic lateral sclerosis (ALS), myasthenia gravis, mononeuropathy, polyneuropathy, spinal muscular atrophy, spinal disorder, acute, transverse myelitis, spinal cord infarction (ischemic myelopathy), intracranial tumor, and spinal tumor; CNS disorders such as Alzheimer's disease, cognitive impairment, stroke, multiple sclerosis, and Parkinson's disease; peripheral artery diseases such as fibromuscular dysplasia, peripheral artery disease (PAD), occlusive thromboangiitis (Buerger's disease), and Kawasaki disease (KD); peripheral venous diseases such as deep vein thrombosis, chronic venous insufficiency, postphlebitic syndrome, and superficial vein thrombosis; and immune dysfunction such as graft versus host Disease (GVHD), secondary immune deficiency, primary immunodeficiency disease, B cell deficiency, T cell deficiency, B and T cell combined deficiency, phagocyte deficiency, complement deficiency.

In one embodiment of the present disclosure, the cell therapeutic agent or pharmaceutical composition may be used to treat and prevent one type of disease selected from the group consisting of myocardial infarction, heart failure, ischemic cardiomyopathy, myocarditis, ischemic enteritis, inflammatory bowel disease, ulcerative colitis, Crohn's disease, acute cholecystitis, acute cholangitis, chronic cholecystitis, acute pancreatitis, chronic pancreatitis, acute nephritis, chronic nephritis, acute renal failure, chronic renal failure, pneumonia, interstitial pneumonia, idiopathic interstitial pneumonia, exfoliative interstitial pneumonia, acute interstitial pneumonia, non-specific interstitial pneumonia, drug-induced lung disease, acute respiratory distress syndrome, chronic obstructive pulmonary disease, cerebral palsy syndrome including pediatric cerebral palsy, amyotrophic lateral sclerosis (ALS), polyneuropathy, spinal muscular atrophy, acute transverse myelitis, stroke, multiple sclerosis, peripheral artery disease (PAD), thromboangiitis obliterans (Buerger's disease), Kawasaki disease (KD), and graft versus host disease (GVHD) in a mammal, but is not limited thereto.

The term "for vascular administration" refers to delivery into the vascular system of a patient. As an example, it may be administration into a blood vessel considered to be a vein, or administration into a blood vessel considered to be an artery. Veins may include internal jugular vein, peripheral vein, coronary vein, hepatic vein, portal vein, great saphenous vein, pulmonary vein, superior vena cava, inferior vena cava, gastric vein, splenic vein, inferior mesenteric vein, superior mesenteric vein, cephalic vein, and/or femoral vein, but are not limited thereto. Arteries may include coronary artery, pulmonary artery, brachial artery, internal carotid artery, aortic arches, femoral artery, peripheral artery, and/or ciliary artery, but are not limited thereto. It may be delivered via the hepatic portal vein, umbilical vein, arteriole or capillary, or to the hepatic portal vein, umbilical vein, arteriole or capillary.

In the present specification, "cell therapeutic agent" is a medicine (US FDA regulations) used for the purposes of treatment, diagnosis, and prevention with cells and tissues prepared through isolation, culture, and special engineering from an individual, and refers to a medicine used for treatment, diagnosis, and prevention purposes through a series of actions such as changing the biological characteristics of cells by ex vivo proliferation selection or other methods of living autologous, allogeneic, or xenogeneic cells to restore cell or tissue function.

The cell therapeutic agent or pharmaceutical composition may be formulated in a suitable form with a pharmaceutical carrier commonly used in cell therapy. The term "pharmaceutically acceptable" refers to a composition that is physiologically acceptable and does not usually cause allergic responses such as gastrointestinal disorders, dizziness, or similar responses when administered to humans. Pharmaceutically acceptable carriers include, for example, water, suitable oils, saline solutions, carriers for parenteral administration such as aqueous glucose and glycol, and may further include stabilizers and preservatives. The suitable stabilizer include antioxidants such as sodium bisulfite, sodium sulfite, or ascorbic acid. Suitable preservative include benzalkonium chloride, methyl- or propyl-paraben, and chlorobutanol. As for other pharmaceutically acceptable carriers, those described in the following literature may be referred to (Remington's Pharmaceutical Sciences, 19th ed., Mack Publishing Company, Easton, PA, 1995).

In addition, the cell therapeutic agent or pharmaceutical composition may be administered by any device capable of moving to target cells.

The cell therapeutic agent or pharmaceutical composition of the present disclosure may include a therapeutically effective amount of the artificially engineered mesenchymal stem cell for the treatment of disease. The term "therapeutically effective amount" refers to an amount of an active ingredient or pharmaceutical composition that induces a biological or medical response in a tissue system, animal, or human, as believed by a researcher, veterinarian, physician or other clinician, and includes an amount that lead to alleviation of the symptoms of the disease or disorder being treated.

It is obvious to those skilled in the art that the content of the artificially engineered mesenchymal stem cell included in the cell therapeutic agent or pharmaceutical composition of the present disclosure will vary depending on the desired effect. Therefore, an optimal cell therapeutic agent content may be easily determined by those skilled in the art, and may be adjusted depending on a variety of factors, including the type of disease, the severity of the disease, the content of other ingredients contained in the composition, the type of formulation, and the patient's age, weight, general health, gender and diet, administration time, administration route and secretion rate of the composition, treatment period, and concurrently used medications. Considering all of the above factors, it is important to include a minimum amount of the maximum effect without side effects. For example, the daily dosage of the stem cells of the present disclosure is $1.0 \times 10^5$ to $1.0 \times 10^{30}$ cells/kg of body weight, preferably $1.0 \times 10^{10}$ to $1.0 \times 10^{20}$ cells/kg of body weight, and may be administered once or in several divided doses. However, it should be understood that the actual dosage of the active ingredient should be determined in light of various related factors such as the disease to be treated, the severity of the disease, the route of administration, the patient's weight, age, and gender, and therefore, the dosage does not limit the scope of the present disclosure in any way.

In addition, the cell therapeutic agent or pharmaceutical composition of the present disclosure may be administered in a manner in the related art through intravascular, i.e. intravenous therapy (i.v) route or intraarterial injection (i.a) route.

The present disclosure provides a method of treatment including administering a therapeutically effective amount of the artificially engineered mesenchymal stem cell of the present disclosure to a mammal having a disease or condition. As used herein, the term "mammal" refers to a mammal that is the subject of treatment, observation or experiment, preferably a human.

The present disclosure also provides a cell therapy method using stem cells, the method including intravascularly administering a therapeutically effective amount of the artificially engineered mesenchymal stem cell to a mammal having a disease or condition.

In one embodiment of the present disclosure, the method can inhibit the blood coagulation mechanism by intravascularly administering the artificially engineered mesenchymal stem cells having reduced or suppressed expression or activity of CD142 by artificially modifying the F3 gene encoding CD142 which is a blood coagulation initiating factor, using genome editing technology.

The present disclosure also provides the use of the artificially engineered mesenchymal stem cell for use in the manufacture of a medicament for inhibiting thrombus formation upon intravascular administration in a mammal having a disease or condition.

In one embodiment of the present disclosure, the disease or condition may be one type of disease selected from the group consisting of myocardial infarction, heart failure, ischemic cardiomyopathy, myocarditis, ischemic enteritis, inflammatory bowel disease, ulcerative colitis, Crohn's disease, acute cholecystitis, acute cholangitis, chronic cholecystitis, acute pancreatitis, chronic pancreatitis, acute nephritis, chronic nephritis, acute renal failure, chronic renal failure, pneumonia, interstitial pneumonia, idiopathic interstitial pneumonia, exfoliative interstitial pneumonia, acute interstitial pneumonia, non-specific interstitial pneumonia, drug-induced lung disease, acute respiratory distress syndrome, chronic obstructive pulmonary disease, cerebral palsy syndrome including pediatric cerebral palsy, amyotrophic lateral sclerosis (ALS), polyneuropathy, spinal muscular atrophy, acute transverse myelitis, stroke, multiple sclerosis, peripheral artery disease (PAD), thromboangiitis obliterans (Buerger's disease), Kawasaki disease (KD), and graft versus host disease (GVHD) in a mammal, and the present disclosure may be used to treat and prevent these diseases, is are not limited thereto.

Hereinafter, the present disclosure will be described in more detail through examples.

These examples are only for illustrating the present disclosure in more detail, and it will be apparent to those skilled in the art that the scope of the present disclosure is not limited by these examples.

**Example 1: Preparation of F3 knocked-out mesenchymal stem cells**

sgRNA design

To knock out the F3 gene (CD142 encoding gene), the CDS region of the human CD142 gene was screened for sgRNA using the Cas-Designer tool. Among the guide sequences predicted at http://www.rgenome.net/cas-designer/, a guide RNA target with a mismatch 0 value of 1 and a mismatch 1,2 value of 0, which is expected to have a small off-target effect, was selected and designed.

The target sequence of the F3 gene is summarized in Table 1 below.

[Table 1]

| Classification | Exon | RGEN Target (5' to 3'direction) with PAM(underlined) | Indel (%) |
|---|---|---|---|
| sgCD142#1 | 1 | GCCCCGAGACCGCCGTCGCTCGG (SEQ ID NO: 1) | 40.4 |
| sgCD142#2 | 1 | TCCGAGCGACGGCGGTCTCGGGG (SEQ ID NO: 2) | 42.2 |
| sgCD142#3 | 1 | GAGCAGGAGCGTCCGAGCGACGG (SEQ ID NO: 3) | 59.9 |
| sgCD142#4 | 1 | CTCGGACGCTCCTGCTCGGCTGG (SEQ ID NO: 4) | 2.5 |
| sgCD142#5 | 1 | TGGGTCTTCGCCCAGGTGGCCGG (SEQ ID NO: 5) | 1 |
| sgCD142#6 | 1 | GCCCAGGTGGCCGGCGCTTCAGG (SEQ ID NO: 6) | 2.9 |
| sgCD142#7 | 1 | GTCCGAGCGACGGCGGTCTCGGG (SEQ ID NO: 7) | 7.7 |
| sgCD142#8 | 1 | CGTCCGAGCGACGGCGGTCTCGG (SEQ ID NO: 8) | 0.6 |
| sgCD142#9 | 2 | GTAGACTTGATTGACGGGTTTGG (SEQ ID NO: 9) | 0 |
| sgCD142#10 | 2 | ACAGTGTAGACTTGATTGACGGG (SEQ ID NO: 10) | 0 |
| sgCD142#11 | 3 | CACATCCTTCACAATCTCGTCGG (SEQ ID NO: 11) | 31.2 |
| sgCD142#12 | 3 | GGATGTGAAGCAGACGTACTTGG (SEQ ID NO: 12) | 40.4 |
| sgCD142#13 | 3 | GGCACGGGTCTTCTCCTACCCGG (SEQ ID NO: 13) | 66.7 |
| sgCD142#14 | 1 | CGGGACCCGGGGCCAGGCAGGGG (SEQ ID NO: 14) | 32.8 |
| sgCD142#15 | 1 | CGCGGGACCCGGGGCCAGGCAGG (SEQ ID NO: 15) | 6.9 |
| sgCD142#17 | 1 | CAGGAGCGTCCGAGCGACGGCGG (SEQ ID NO: 16) | 23.4 |
| sgCD142#19 | 1 | GTCGCTCGGACGCTCCTGCTCGG (SEQ ID NO: 17) | 27.8 |
| sgCD142#20 | 1 | TCGGACGCTCCTGCTCGGCTGGG (SEQ ID NO: 18) | 27.9 |
| sgCD142#21 | 2 | TGGCAGCATATAATTTAACTTGG (SEQ ID NO: 19) | 0 |
| sgCD142#22 | 2 | TAGACTTGATTGACGGGTTTGGG (SEQ ID NO: 20) | 0 |
| sgCD142#23 | 3 | CCTCACCGACGAGATTGTGAAGG (SEQ ID NO: 21) | 53.4 |
| sgCD142#24 | 3 | CCTTCACAATCTCGTCGGTGAGG (SEQ ID NO: 22) | 0.9 |

(continued)

| Classification | Exon | RGEN Target (5' to 3'direction) with PAM(underlined) | Indel (%) |
|---|---|---|---|
| sgCD142#25 | 3 | TGAAGCAGACGTACTTGGCACGG (SEQ ID NO: 23) | 16.8 |
| sgCD142#26 | 3 | GAAGCAGACGTACTTGGCACGGG (SEQ ID NO: 24) | 19.3 |
| sgCD142#27 | 3 | CGGGTCTTCTCCTACCCGGCAGG (SEQ ID NO: 25) | 2.5 |
| sgCD142#28 | 3 | CTCCTACCCGGCAGGGAATGTGG (SEQ ID NO: 26) | 7.5 |
| sgCD142#29 | 3 | GTGCTCTCCACATTCCCTGCCGG (SEQ ID NO: 27) | 12 |
| sgCD142#30 | 3 | GTGGAGAGCACCGGTTCTGCTGG (SEQ ID NO: 28) | 4.7 |
| sgCD142#31 | 3 | TGGAGAGCACCGGTTCTGCTGGG (SEQ ID NO: 29) | 43.2 |
| sgCD142#32 | 3 | GGAGAGCACCGGTTCTGCTGGGG (SEQ ID NO: 30) | 83.6 |
| sgCD142#33 | 3 | TCTGGGGAGTTCTCATACAGAGG (SEQ ID NO: 31) | 81.5 |
| sgCD142#34 | 3 | CAGGTAAGGTGTGAACTCTGGGG (SEQ ID NO: 32) | 57.1 |
| sgCD142#35 | 3 | CCAGGTAAGGTGTGAACTCTGGG (SEQ ID NO: 33) | 11.3 |
| sgCD142#36 | 3 | TCCAGGTAAGGTGTGAACTCTGG (SEQ ID NO: 34) | 8.7 |
| sgCD142#37 | 4 | AACAATTCAGAGTTTTGAACAGG (SEQ ID NO: 35) | 7.4 |
| sgCD142#3 8 | 4 | ATGTGACCGTAGAAGATGAACGG (SEQ ID NO: 36) | 23.8 |
| sgCD142#39 | 4 | TAAAGTCCGTTCATCTTCTACGG (SEQ ID NO: 37) | 3.5 |
| sgCD142#40 | 4 | ATGAACGGACTTTAGTCAGAAGG (SEQ ID NO: 38) | 0.3 |
| sgCD142#41 | 4 | ACAACACTTTCCTAAGCCTCCGG (SEQ ID NO: 39) | 1.1 |
| sgCD142#42 | 4 | CAACACTTTCCTAAGCCTCCGGG (SEQ ID NO: 40) | 4.7 |
| sgCD142#45 | 6 | ATTCTACATCATTGGAGCTGTGG (SEQ ID NO: 41) | 0.2 |
| sgCD142#46 | 6 | CATTGGAGCTGTGGTATTTGTGG (SEQ ID NO: 42) | 0 |
| sgCD142#47 | 6 | ACTTGTGTAGAGATATAGCCAGG (SEQ ID NO: 43) | 2.0 |

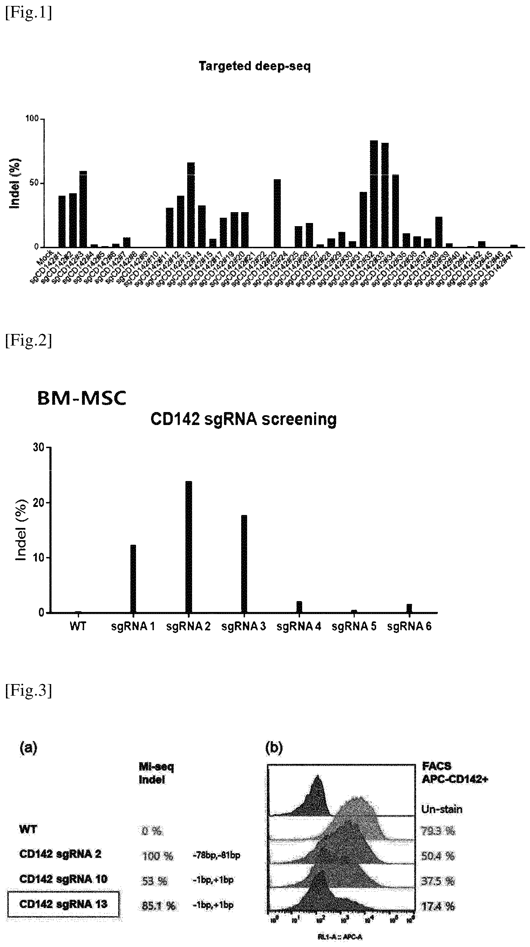

Each guide RNA targeting the target sequences of SEQ ID NOs: 1 to 43 was synthesized and used in subsequent experiments. The indel efficiency of each target sequence for each guide RNA was measured using the targeted deep sequencing method below, and the results are shown in FIGS. 1 and 2.
Guide RNA was transcribed in vitro using the MEGA short script T7 kit (Ambion) according to the manufacturer's instructions. A template for sgRNA was prepared through annealing and extension of two complementary oligonucleotides.

Ribonucleoprotein (RNP) delivery

A RNP complex were introduced by electroporation using a 4D-Nucleofector (Lonza). Specifically, the RNP complex was formed by mixing 4 ug of Cas9 protein and 4 ug of in vitro transcribed sgRNA (prepared according to the manufacturer's protocol using T7 polymerase (New England BioLabs)), and the mixture was incubated for 10 min at room temperature. The RNP complex was electroporated using the nucleofector program EW-104 together with 20 ul of Primary P1 buffer-treated each $4 \times 10^5$ Human bone marrow MSC (Lonza, Cat. No. PT-2501) and umbilical cord MSC (ATCC, Cat. No. PCS-500-010). As a result, umbilical cord blood-derived mesenchymal stem cells with the F3 gene knocked out (F3 KO UC-MSC) and bone marrow-derived mesenchymal stem cells with the F3 gene knocked out (F3 KO BM-MSC)) were obtained, respectively.

Targeted deep sequencing

Genomic DNA (gDNA) was extracted from the obtained F3 KO UC-MSC and F3 KO BM-MSC using a blood genomic DNA extraction kit (Favorgen) according to the manufacturer's protocol. To amplify a target region, 100 ng of genomic DNA (gDNA) was amplified using Phusion high-hidelity DNA polymerase PCR Polymerase (NEB). For deep sequencing library generation, amplicons were amplified once more using TruSeq HT dual index primers (Illumina, San Diego, CA, USA). Paired-end sequencing was performed using the Illumina Miniseq System, and the indel frequency was calculated at 'http://www.rgenome.net/'. The primer sequences for each target sequence used for targeted deep sequencing are shown in Table 2.

[Table 2]

| Classification | Forward primer (5'-3') | Reverse primer (5'-3') |
|---|---|---|
| sgCD142#1 | | |
| sgCD142#2 | | |
| sgCD142#3 | | |
| sgCD142#4 | | |
| sgCD142#5 | | |
| sgCD142#6 | | |
| sgCD142#7 | ttcagcccaacctccccagc (SEQ ID NO: 44) | cccgctgccagccaggactg (SEQ ID NO: 45) |
| sgCD142#8 | | |
| sgCD142#14 | | |
| sgCD142#15 | | |
| sgCD142#17 | | |
| sgCD142#19 | | |
| sgCD142#20 | | |
| sgCD142#9 | | |
| sgCD142#10 | atacatcaatacattcgagtgc (SEQ ID NO: 46) | cttgttcagcataggacag (SEQ ID NO: 47) |
| sgCD142#21 | | |
| sgCD142#22 | | |
| sgCD142#11 | | |
| sgCD142#12 | | |
| sgCD142#13 | | |
| sgCD142#23 | | |
| sgCD142#24 | | |
| sgCD142#25 | | |
| sgCD142#26 | | |
| sgCD142#27 | gcactaagtcaggagattgg (SEQ ID NO: 48) | gttcagacgtttctaacaag (SEQ ID NO: 49) |
| sgCD142#28 | | |
| sgCD142#29 | | |
| sgCD142#30 | | |
| sgCD142#31 | | |
| sgCD142#32 | | |
| sgCD142#33 | | |
| sgCD142#34 | | |
| sgCD142#35 | | |
| sgCD142#36 | | |

(continued)

| Classification | Forward primer (5'-3') | Reverse primer (5'-3') |
|---|---|---|
| sgCD142#37 | | |
| sgCD142#38 | | |
| sgCD142#39 | ctttgtacagaattctttgg (SEQ ID NO: 50) | tgctcacctttcctgaac (SEQ ID NO: 51) |
| sgCD142#40 | | |
| sgCD142#41 | | |
| sgCD142#42 | | |
| sgCD142#45 | ccactgataaatgggatttgag (SEQ ID NO: 52) | atagcatttgcagtagctcc (SEQ ID NO: 53) |

As a result of targeted deep sequencing, the mutation location of F3 KO US-MSC for each target sequence was indicated based on the sequence of a wild-type F3 gene. As an example, the results of targeted deep sequencing of SEQ ID NOs: 1 to 42 were shown in FIG. 1.

The results of analyzing the indel frequency in bone marrow-derived mesenchymal stem cells (F3 KO BM-MSC) in which the F3 gene was knocked out using the screened F3 target sgRNA through targeted deep sequencing are shown in FIG. 2.

FACS analysis

After washing F3 KO UC-MSCs twice with phosphate buffer saline (PBS), cells were removed from the culture plate using 0.05% trypsin-EDTA and centrifuged at 1,000 rpm for 5 minutes. Cells were suspended in 100 ul of FACS staining buffer and anti-CD142 antibody (Biolegend, Cat No. 365206) was mixed and reacted at 4 degrees for 20 min, washed twice with FACS staining buffer, and suspended in 300 ul of PBS for FACS analysis.

FIG. 3 is a graph showing the indel frequency in umbilical cord blood-derived mesenchymal stem cells (F3 KO UC-MSC) in which the F3 gene was knocked out using the screened F3 target sgRNA (FIG. 3(a)), and the level of CD142 expression on the surface of umbilical cord blood-derived mesenchymal stem cells (F3 KO UC-MSC) through FACS analysis in which the F3 gene was knocked out (FIG. 3(b)). Referring to FIG. 3, compared to wild-type stem cells, it was confirmed that the expression of CD142 on the cell surface of umbilical cord blood-derived mesenchymal stem cells in which the F3 gene was knocked out was reduced.

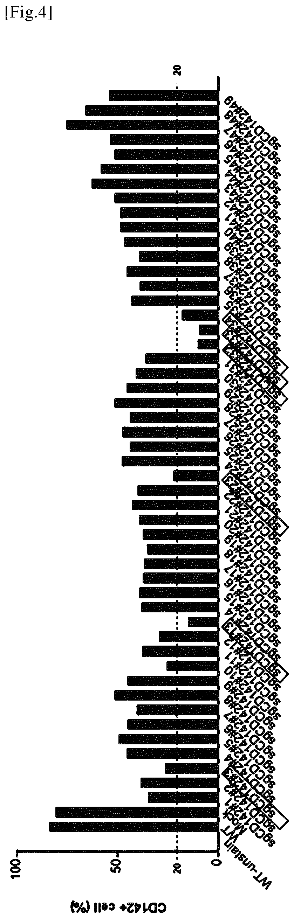

FIG. 4 is a graph showing the level of CD142 expression on the surface of umbilical cord blood-derived mesenchymal stem cells (F3 KO UC-MSC) in which the F3 gene has been knocked out through FACS analysis using each guide RNA targeting the target sequence of SEQ ID NOs. 1 to 43 of the F3 gene. As shown in FIG. 4, umbilical cord blood-derived mesenchymal stem cells in which the F3 gene was knocked out had reduced expression of CD142 on the cell surface compared to wild-type stem cells, especially when targeting the target sequence of SEQ ID NO: 3 (sgCD142#3), SEQ ID NO: 13 (sgCD142#13), SEQ ID NO: 21 (sgCD142#23), SEQ ID NO: 30 (sgCD142#32), SEQ ID NO: 31 (sgCD142#33), and SEQ ID NO: 32 (sgCD142#34), the expression of CD142 on the cell surface was significantly reduced.

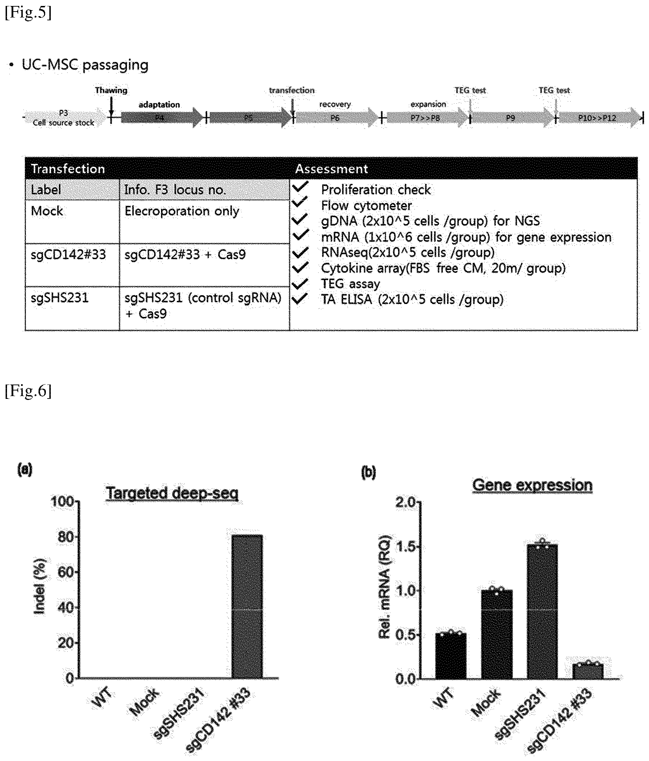

FIG. 5 shows an experimental schedule according to passage of umbilical cord blood-derived mesenchymal stem cells (US-MSC) in an experimental example according to an embodiment of the present disclosure.

FIG. 6 is a graph showing the results of measuring indel efficiency through a targeted deep sequencing method (FIG. 6(a)); and F3 mRNA expression level (FIG. 6(b)) for wild-type umbilical cord blood-derived stem cells (WT), umbilical cord blood-derived stem cells with electroporation alone and no gene engineering (Mock), and umbilical cord blood-derived stem cells with knockout of the target sequence of SEQ ID NO: 31 (sgCD142#33).

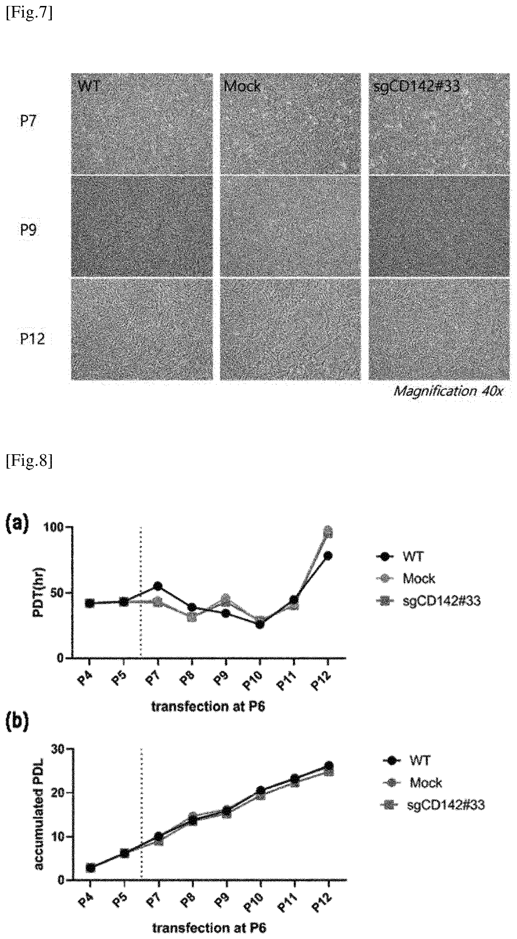

FIG. 7 shows the results of microscopic observation of the morphology of mesenchymal stem cells after transfection. As shown in FIG. 7, there was no difference in morphology between wild-type stem cells (WT) and mesenchymal stem cells (Mock, sgCD142#33) transfected by electroporation, and all were P12. showed senescence.

Population doubling level (PDL) and Population doubling time (PDT)

To determine the growth rate of cells, the number of cells was measured before and after passaged culture. The value obtained by dividing the harvest cell count (Ct) by the seeding cell count ($C_i$) was regarded as the growth rate, and PDL (n) was calculated using the following Expression:

[Expression 1]

$$2^{n}=\frac{C_t}{C_i}$$

The culture time (hr) divided by PDL was expressed as population doubling time (PDT).

[Expression 2]

$$Population\ Doubling\ Time(hr) = \frac{culture\ duration(hr)}{\log(2)\frac{C_t}{C_i}}$$

FIG. 8 is a graph showing population doubling level (PDL) (FIG. 8(a)) and population doubling time (PDT) (FIG. 8(b)) of wild-type umbilical cord blood-derived mesenchymal stem cells (WT), umbilical cord blood-derived mesenchymal stem cells with electroporation alone and no gene engineering (Mock), and umbilical cord blood-derived stem cells with knockout of the target sequence of SEQ ID NO: 31(sgCD142#33). As shown in FIG. 8, there was little difference in population doubling level (PDL) and population doubling time (PDT) in all groups.

Flow cytometer analysis

Cultured umbilical cord blood-derived stem cells (UC-MSC) were washed twice with phosphate buffer saline (PBS), then cells were removed using 0.05% trypsin-EDTA and centrifuged at 1,500 rpm for 5 minutes. For each group, $1\times10^6$ cells were suspended in 100 ul of FACS staining buffer (2% FBS in PBS), antibodies (CD142, CD34, CD45, CD73, CD90, CD105, CD29, CD51, CD44, CD51/61) were mixed and reacted at 4 degrees for 20 minutes, washed twice with FACS staining buffer, suspended in 300 ul of PBS, and flow cytometer analysis was performed. The median fluorescence intensity (MFI) obtained from this was performed. ), the CD142 expression level was measured.

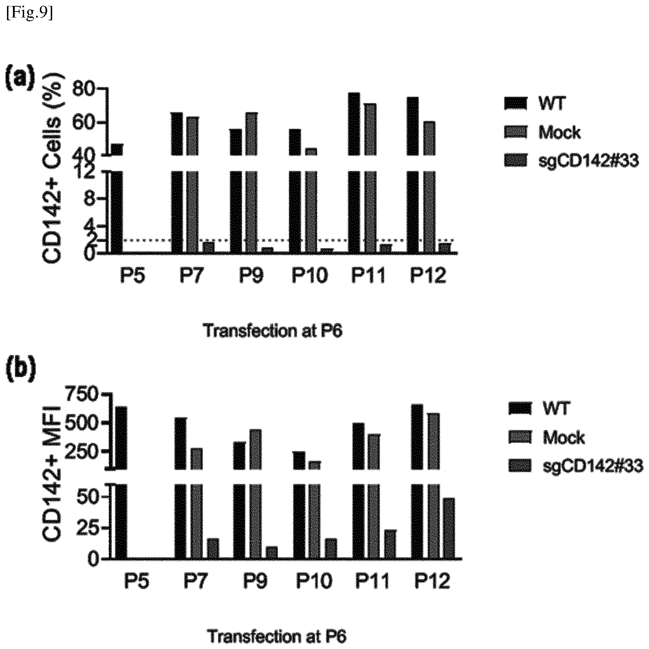

FIG. 9 is a graph showing the percentage of cells showing CD142 expression (FIG. 9(a)) and CD142 median fluorescence intensity (MFI) (FIG. 9(b)) of wild-type umbilical cord blood-derived mesenchymal stem cells (WT), umbilical cord blood-derived mesenchymal stem cells with electroporation alone and no gene engineering (Mock), and umbilical cord blood-derived stem cells with knockout of the target sequence of SEQ ID NO: 31 (sgCD142#33). As shown in FIG. 9, the percentage of cells showing CD142 expression and the average fluorescence intensity of CD142 were found to be reduced in umbilical cord blood-derived stem cells with knockout of the target sequence of SEQ ID NO: 31 (sgCD142#33).

Thromboelastrometry test

Cultured cord blood-derived mesenchymal stem cells (UC-MSC) were harvested, $4\times10^5$ cells were prepared for each group, and reacted with 400 ul of fresh human whole blood prepared in a sterilized tube. The well-mixed cells and whole blood sample were immediately taken to a test equipment to measure thromboelastrometry. A TEG 6s equipment was used to measure thromboelastrometry, after inserting the kit to check thromboelastrometry into the equipment, the stem cell sample mixed with whole blood was mixed well and placed in the sample loading chamber to check the response.
FIG. 10 is a graph showing coagulation response time (R-time: time from the start of analysis until the thrombus amplitude reaches 2 mm) (FIG. 10(a)), thrombus dynamics (K-time: time from when the thrombus amplitude reaches 2 mm until the amplitude reaches 20 mm) (FIG. 10(b)), thrombus formation intensity (alpha angle: the angle formed by the tangent at the midpoint of R and K times) (FIG. 10(c)), and thrombus maximum amplitude (MA: absolute thrombus strength) (FIG. 10(d)) of wild-type umbilical cord blood-derived stem cells (WT), umbilical cord blood-derived stem cells with electroporation alone and no gene engineering (Mock), and umbilical cord blood-derived stem cells with knockout of the target sequence of SEQ ID NO: 31 (sgCD142#33), when measuring using TEG 6s equipment. Referring to FIG. 10, the coagulation time of human blood cultured with wild-type cord blood-derived stem cells (WT) was decreased, while the blood coagulation time of human blood cultured with umbilical cord blood-derived stem cells with knockout of the target sequence of SEQ ID NO: 31 (sgCD142#33) was restored to normal.

Enzyme-linked immunosorbent assay (ELSIA)

Cultured umbilical cord blood-derived stem cells (UC-MSC) were harvested and tissue factor activity was confirmed. This was confirmed using Abcam's Tissue factor Activity assay kit. For each group, $2 \times 10^5$ cells were lysed using 500 ul of 50 mM Tris buffered saline including Triton x-100 or 0.1% Tween 20 by incubating the cells on ice for 30 minutes. The procedure was carried out according to the kit protocol, and activity was confirmed.

FIG. 11 is a graph showing tissue factor in cell lysate (FIG. 11(a)) and tissue factor in conditioned media (FIG. 11(b)) of wild-type umbilical cord blood-derived stem cells (WT), umbilical cord blood-derived stem cells with electroporation alone and no gene engineering (Mock), and umbilical cord blood-derived stem cells with knockout of the target sequence of SEQ ID NO: 31 (sgCD142#33). As shown in FIG. 11, umbilical cord blood-derived stem cells with knockout of the target sequence of SEQ ID NO: 31 (sgCD142#33) showed the lowest tissue factor activity in cell lysate and conditioned media.

RNA-seq (QuantSeq)

Total RNA was isolated using Trizol reagent (Invitrogen). RNA quality was measured using an Agilent 2100 bioanalyzer using the RNA 6000 Nano Chip (Agilent Technologies, Amstelveen, The Netherlands), and an amount of RNA was quantified using an ND-2000 Spectrophotometer (Thermo Inc., DE, USA).

For control and sample RNA measurements, library construction was performed using the QuantSeq 3' mRNA-Seq Library Prep Kit (Lexogen, Inc., Austria) according to the manufacturer's instructions. 500 ng of total RNA was prepared and reverse transcribed by hybridizing an oligo-dT primer including an Illumina platform compatible sequence at the 5' end to the RNA. After RNA template digestion, second-strand synthesis was initiated using random primers including an Illumine-compatible linker sequence at the 5' end. Magnetic beads were used to purify the double-stranded library. The entire adapter sequence for cluster generation was added to amplify the library. It was purified from the completed double stranded library PCR components. This was followed by a high-throughput sequencing procedure (single-end 75 sequence) (NextSeq 500, Illumina Inc.). QuantSeq 3'mRNA-Seq reads were aligned using Bowtie2 (Langmead and Salzberg, 2012). A Bowtie2 index was generated from genome assembly sequences or transcripts. The alignment file was used to assemble transcripts, estimate abundance, and detect differential expression of genes. Differentially expressed genes were determined based on counts from unique and multiple alignments using coverage in Badtools (Quinlan AR, 2010). Using Bioconductor (Gentleman et al., 2004), RC (Read Count) data was processed based on a TMM+CPM normalization method using EdgeR within R (R development Core Team, 2020). Gene classification was based on searches by the DAVID (http://david.abcc.ncifcrf.gov/) and Medline databases (http://www.ncbi.nlm.nih.gov/) engines.

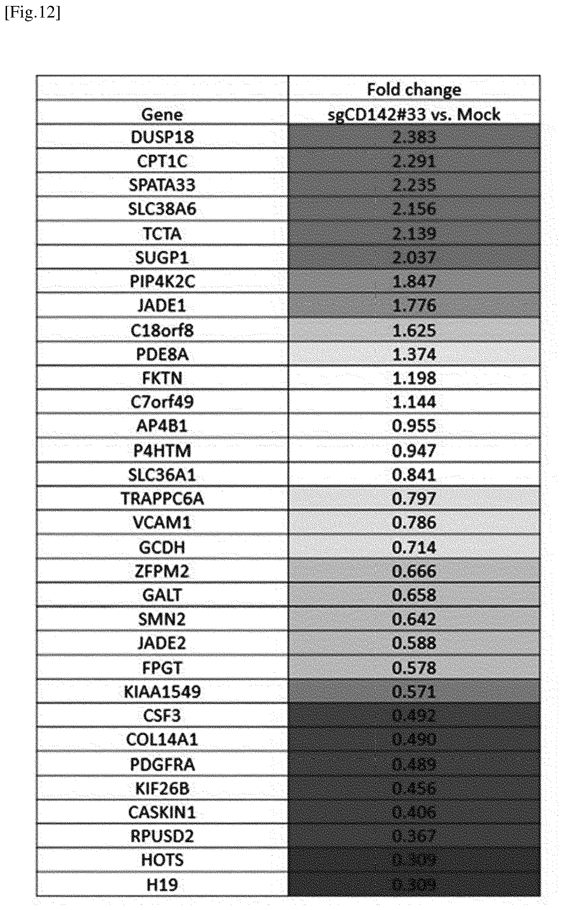

FIG. 12 is a table showing a list of genes with a p-value of less than 0.05 and a normalized data (log 2) value of 4 or more as a result of RNA-seq (QuantSeq) analysis.

Antibody microarray

Antibody array analysis of gene-edited mesenchymal stem cells was performed by Full Moon Biosystems. Cell lysate was prepared using Protein Extraction Kit (Full Moon BioSystems). The clear supernatant of the lysate was isolated, biotinylated, and incubated using an antibody microarray (Phospho Explorer Antibody Arrays, Full Moon BioSystems) of phosphorylated proteins for 2 hours at room temperature. Array slides were washed with wash buffer (Full Moon BioSystems) and rinsed with deionized water. Slides were then incubated with Cy3-Streptavidin for 45 min at room temperature and then washed, rinsed, and dried. Arrays were scanned on a GenePix Array Scanner (Molecular Devices). Image quantification was performed with GenePix Pro (Molecular Devices). Signal intensity data for each point on the array was extracted from the array image. Since two replicates are printed for each antibody, the average signal intensity of the replicates is determined. Data are then normalized to the median (signal intensity) of all antibodies on the slide. Lastly, the fold change between control and treated samples was determined using normalized data (signal of treated samples divided by that of control samples).

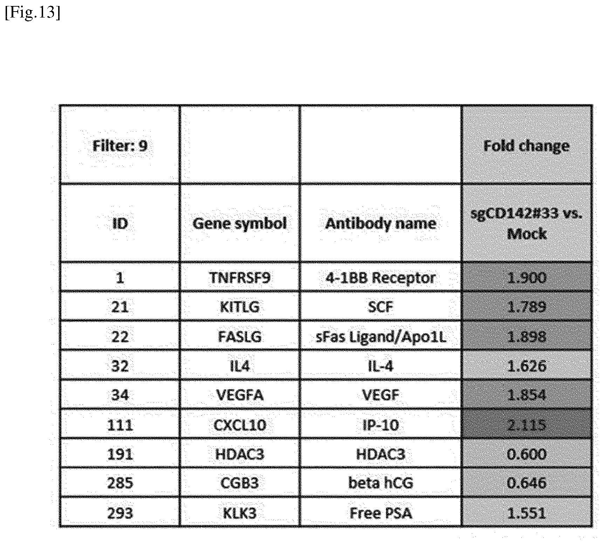

FIG. 13 is a table showing the results of secretome analysis through antibody microarray.

**Claims**

1. Artificially engineered mesenchymal stem cells comprising an artificially engineered F3 gene, wherein the artificially engineered F3 gene is different from the F3 gene sequence of a wild-type mesenchymal stem cell, the artificially engineered F3 gene includes one or more indels in the nucleic acid sequence, and an expression level of CD142 on the surface of the artificially engineered mesenchymal stem cell is reduced compared to the wild-type mesenchymal stem cell, thereby improving hemocompatibility.

2. The artificially engineered mesenchymal stem cell of claim 1, wherein the indel is located within a protospacer-adjacent motif (PAM) sequence in the first, second, third, fourth or sixth exon region of the F3 gene, or within a contiguous sequence of 5 to 50 nucleotides adjacent to the 5' or 3' end of the PAM sequence.

3. The artificially engineered mesenchymal stem cell of claim 1, wherein a sequence of the artificially engineered F3 gene does not include one or more sequences selected from the group consisting of SEQ ID NOs: 1 to 43.

4. The artificially engineered mesenchymal stem cell of claim 1, wherein, in the artificially engineered mesenchymal stem cell, an mRNA transcribed from the artificially engineered F3 gene has a lower mRNA expression level or a different sequence compared to the mRNA expression level transcribed from the F3 gene of the wild-type mesenchymal stem cell.

5. The artificially engineered mesenchymal stem cell of claim 1, wherein the artificially engineered mesenchymal stem cells is derived from fat, bone marrow, umbilical cord, placenta, amniotic fluid, amniotic membrane, tissue, umbilical cord blood, or perinatal tissue.

6. A composition for preparing hemocompatible mesenchymal stem cells, the composition comprising:

   a guide nucleic acid including a guide sequence capable of targeting a target sequence of the F3 gene of a mesenchymal stem cell, or a nucleic acid encoding the same; and
   an editor protein or a nucleic acid encoding the same.

7. The composition of claim 6, wherein the target sequence is one or more sequences selected from SEQ ID NO: 1 to SEQ ID NO: 43.

8. The composition of claim 6, wherein the composition includes the editor protein and the guide nucleic acid in the form of ribonucleoprotein (RNP).

9. The composition of claim 6, wherein the composition includes a nucleic acid encoding the editor protein and a nucleic acid encoding the guide nucleic acid in the form of one or more vectors.

10. The composition of claim 9, wherein the vector is selected from the group consisting of plasmid, retrovirus, lentivirus, adenovirus, adeno-associated virus, vaccinia virus, poxvirus, and herpes simplex virus.

11. A preparation method for hemocompatible stem cells, the method comprising:

   (1) introducing a composition for preparing hemocompatible mesenchymal stem cells including a guide nucleic acid capable of targeting a target sequence of F3 gene or a nucleic acid encoding the same; and an editor protein or a nucleic acid encoding the same into isolated mesenchymal stem cells; and
   (2) editing the F3 gene to reduce or suppress the expression or activity of CD142 by generating an indel in the target sequence of the F3 gene located in the genome of the mesenchymal stem cell.

12. The method of 11, wherein the target sequence is one or more sequences selected from SEQ ID NO: 1 to SEQ ID NO: 43.

13. The method of 11, wherein the composition includes a nucleic acid encoding the editor protein and a nucleic acid encoding the guide sequence in the form of one or more vectors.

14. The method of 11, wherein a CRISPR/Cas9 complex including Streptococcus pyogenes-derived Cas9 protein and guide RNA that may target the target sequence of the F3 gene is contacted with the target sequence of F3 gene located within the genome of the mesenchymal stem cell, thereby generating an indel within the target sequence.

15. A cell therapeutic agent for vascular administration comprising the artificially engineered mesenchymal stem cells of any one of claims 1 to 5 as an active ingredient.

16. The cell therapeutic agent of claim 15, wherein the cell therapeutic agent for vascular administration is intended to treat one type of disease selected from the group consisting of myocardial infarction, heart failure, ischemic cardiomyopathy, myocarditis, ischemic enteritis, inflammatory bowel disease, ulcerative colitis, Crohn's disease, acute cholecystitis, acute cholangitis, chronic cholecystitis, acute pancreatitis, chronic pancreatitis, acute nephritis, chronic nephritis, acute renal failure, chronic renal failure, pneumonia, interstitial pneumonia, idiopathic interstitial pneumonia, exfoliative interstitial pneumonia, acute interstitial pneumonia, non-specific interstitial pneumonia, drug-induced lung disease, acute respiratory distress syndrome, chronic obstructive pulmonary disease, cerebral palsy syndrome including pediatric cerebral palsy, amyotrophic lateral sclerosis (ALS), polyneuropathy, spinal muscular atrophy, acute transverse myelitis, stroke, multiple sclerosis, peripheral artery disease (PAD), thromboangiitis obliterans (Buerger's disease), Kawasaki disease (KD), and graft versus host disease (GVHD).

17. A pharmaceutical composition comprising the artificially engineered mesenchymal stem cells of any one of claims 1 to 5 as an active ingredient.

18. The pharmaceutical composition of claim 17, wherein the composition is administered intravascularly.

19. The pharmaceutical composition of claim 17, wherein the composition is intended for use in the prevention or treatment of one type of disease selected from the group consisting of myocardial infarction, heart failure, ischemic cardiomyopathy, myocarditis, ischemic enteritis, inflammatory bowel disease, ulcerative colitis, Crohn's disease, acute cholecystitis, acute cholangitis, chronic cholecystitis, acute pancreatitis, chronic pancreatitis, acute nephritis, chronic nephritis, acute renal failure, chronic renal failure, pneumonia, interstitial pneumonia, idiopathic interstitial pneumonia, exfoliative interstitial pneumonia, acute interstitial pneumonia, non-specific interstitial pneumonia, drug-induced lung disease, acute respiratory distress syndrome, chronic obstructive pulmonary disease, cerebral palsy syndrome including pediatric cerebral palsy, amyotrophic lateral sclerosis (ALS), polyneuropathy, spinal muscular atrophy, acute transverse myelitis, stroke, multiple sclerosis, peripheral artery disease (PAD), thromboangiitis obliterans (Buerger's disease), Kawasaki disease (KD), and graft versus host disease (GVHD).

20. A cell therapy method using stem cells, the method comprising intravascularly administering a therapeutically effective amount of the artificially engineered mesenchymal stem cell of any one of claims 1 to 5 to a mammal having a disease or condition.

21. The method of claim 20, wherein the disease or condition is one selected from the group consisting of myocardial infarction, heart failure, ischemic cardiomyopathy, myocarditis, ischemic enteritis, inflammatory bowel disease, ulcerative colitis, Crohn's disease, acute cholecystitis, acute cholangitis, chronic cholecystitis, acute pancreatitis, chronic pancreatitis, acute nephritis, chronic nephritis, acute renal failure, chronic renal failure, pneumonia, interstitial pneumonia, idiopathic interstitial pneumonia, exfoliative interstitial pneumonia, acute interstitial pneumonia, non-specific interstitial pneumonia, drug-induced lung disease, acute respiratory distress syndrome, chronic obstructive pulmonary disease, cerebral palsy syndrome including pediatric cerebral palsy, amyotrophic lateral sclerosis (ALS), polyneuropathy, spinal muscular atrophy, acute transverse myelitis, stroke, multiple sclerosis, peripheral artery disease (PAD), thromboangiitis obliterans (Buerger's disease), Kawasaki disease (KD), and graft versus host disease (GVHD).

22. A use of the artificially engineered mesenchymal stem cell of any one of claims 1 to 5 for use in the manufacture of a medicament for inhibiting thrombus formation upon intravascular administration in a mammal having a disease or condition.

23. The use of claim 22, wherein the disease or condition is one selected from the group consisting of myocardial infarction, heart failure, ischemic cardiomyopathy, myocarditis, ischemic enteritis, inflammatory bowel disease, ulcerative colitis, Crohn's disease, acute cholecystitis, acute cholangitis, chronic cholecystitis, acute pancreatitis, chronic pancreatitis, acute nephritis, chronic nephritis, acute renal failure, chronic renal failure, pneumonia, interstitial pneumonia, idiopathic interstitial pneumonia, exfoliative interstitial pneumonia, acute interstitial pneumonia, non-specific interstitial pneumonia, drug-induced lung disease, acute respiratory distress syndrome, chronic obstructive pulmonary disease, cerebral palsy syndrome including pediatric cerebral palsy, amyotrophic lateral sclerosis (ALS), polyneuropathy, spinal muscular atrophy, acute transverse myelitis, stroke, multiple sclerosis, peripheral artery disease (PAD), thromboangiitis obliterans (Buerger's disease), Kawasaki disease (KD), and graft versus host disease (GVHD).

[Fig.1]

**Targeted deep-seq**

[Fig.2]

**BM-MSC**

**CD142 sgRNA screening**

[Fig.3]

| (a) | Mi-seq Indel | | (b) | FACS APC-CD142+ |
|---|---|---|---|---|
| | | | | Un-stain |
| WT | 0 % | | | 79.3 % |
| CD142 sgRNA 2 | 100 % | -78bp,-81bp | | 50.4 % |
| CD142 sgRNA 10 | 53 % | -1bp,+1bp | | 37.5 % |
| CD142 sgRNA 13 | 85.1 % | -1bp,+1bp | | 17.4 % |

[Fig.4]

[Fig.5]

• UC-MSC passaging

| Transfection | | Assessment |
|---|---|---|
| Label | Info. F3 locus no. | ✓ Proliferation check |
| Mock | Elecroporation only | ✓ Flow cytometer |
| | | ✓ gDNA (2x10^5 cells /group) for NGS |
| | | ✓ mRNA (1x10^6 cells /group) for gene expression |
| sgCD142#33 | sgCD142#33 + Cas9 | ✓ RNAseq(2x10^5 cells /group) |
| | | ✓ Cytokine array(FBS free CM, 20m/ group) |
| | | ✓ TEG assay |
| sgSHS231 | sgSHS231 (control sgRNA) + Cas9 | ✓ TA ELISA (2x10^5 cells /group) |

[Fig.6]

(a) Targeted deep-seq

(b) Gene expression

[Fig.7]

Magnification 40x

[Fig.8]

[Fig.9]

(a)

Transfection at P6

(b)

Transfection at P6

[Fig.10]

[Fig.11]

[Fig.12]

| Gene | Fold change sgCD142#33 vs. Mock |
|---|---|
| DUSP18 | 2.383 |
| CPT1C | 2.291 |
| SPATA33 | 2.235 |
| SLC38A6 | 2.156 |
| TCTA | 2.139 |
| SUGP1 | 2.037 |
| PIP4K2C | 1.847 |
| JADE1 | 1.776 |
| C18orf8 | 1.625 |
| PDE8A | 1.374 |
| FKTN | 1.198 |
| C7orf49 | 1.144 |
| AP4B1 | 0.955 |
| P4HTM | 0.947 |
| SLC36A1 | 0.841 |
| TRAPPC6A | 0.797 |
| VCAM1 | 0.786 |
| GCDH | 0.714 |
| ZFPM2 | 0.666 |
| GALT | 0.658 |
| SMN2 | 0.642 |
| JADE2 | 0.588 |
| FPGT | 0.578 |
| KIAA1549 | 0.571 |
| CSF3 | 0.492 |
| COL14A1 | 0.490 |
| PDGFRA | 0.489 |
| KIF26B | 0.456 |
| CASKIN1 | 0.406 |
| RPUSD2 | 0.367 |
| HOTS | 0.309 |
| H19 | 0.309 |

[Fig.13]

| Filter: 9 | | | Fold change |
|---|---|---|---|
| ID | Gene symbol | Antibody name | sgCD142#33 vs. Mock |
| 1 | TNFRSF9 | 4-1BB Receptor | 1.900 |
| 21 | KITLG | SCF | 1.789 |
| 22 | FASLG | sFas Ligand/Apo1L | 1.898 |
| 32 | IL4 | IL-4 | 1.626 |
| 34 | VEGFA | VEGF | 1.854 |
| 111 | CXCL10 | IP-10 | 2.115 |
| 191 | HDAC3 | HDAC3 | 0.600 |
| 285 | CGB3 | beta hCG | 0.646 |
| 293 | KLK3 | Free PSA | 1.551 |

<table>
<tr><td colspan="2" align="center">**INTERNATIONAL SEARCH REPORT**</td><td colspan="2">International application No.<br><br>**PCT/KR2022/011139**</td></tr>
</table>

**A. CLASSIFICATION OF SUBJECT MATTER**

**C12N 5/0775**(2010.01)i; **C07K 14/705**(2006.01)i; **C12N 15/10**(2006.01)i; **C12N 15/113**(2010.01)i; **C12N 15/90**(2006.01)i; **C12N 9/22**(2006.01)i; **A61K 48/00**(2006.01)i; **A61K 35/28**(2006.01)i

According to International Patent Classification (IPC) or to both national classification and IPC

**B. FIELDS SEARCHED**

Minimum documentation searched (classification system followed by classification symbols)

C12N 5/0775(2010.01); A61K 47/68(2017.01); C07K 16/30(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: F3, Tissue Factor, CD142, 중간엽 줄기세포(mesenchymal stem cell), 인델(indel), CRISPR, 세포 치료제(cell therapy)

**C. DOCUMENTS CONSIDERED TO BE RELEVANT**

| Category* | Citation of document, with indication, where appropriate, of the relevant passages | Relevant to claim No. |
|---|---|---|
| Y | RANGASAMI, Vignesh K. et al. Pluronic micelle-mediated tissue factor silencing enhances hemocompatibility, stemness, differentiation potential, and paracrine signaling of mesenchymal stem cells. Biomacromolecules. 05 April 2021, vol. 22, pp. 1980-1989.<br>     See abstract; and pages 1980 and 1987. | 1-19,22-23 |
| Y | ROSELL, Axel et al. Evaluation of different commercial antibodies for their ability to detect human and mouse tissue factor by western blotting. Research and Practice in Thrombosis and Haemostasis. 2020, vol. 4, pp. 1013-1023.<br>     See page 1015. | 1-19,22-23 |
| A | MOLL, Guido et al. Intravascular mesenchymal stromal/stem cell therapy product diversification: time for new clinical guidelines. Trends in Molecular Medicine. 2019, vol. 25, no. 2, pp. 149-163.<br>     See entire document. | 1-19,22-23 |
| A | OELLER, Michaela et al. Selection of tissue factor-deficient cell transplants as a novel strategy for improving hemocompatibility of human bone marrow stromal cells. Theranostics. 2018, vol. 8, no. 5, pp. 1421-1434.<br>     See entire document. | 1-19,22-23 |

☑ Further documents are listed in the continuation of Box C.  ☑ See patent family annex.

| | |
|---|---|
| \*     Special categories of cited documents:<br>"A"   document defining the general state of the art which is not considered to be of particular relevance<br>"D"   document cited by the applicant in the international application<br>"E"   earlier application or patent but published on or after the international filing date<br>"L"   document which may throw doubts on priority claim(s) or which is cited to establish the publication date of another citation or other special reason (as specified)<br>"O"   document referring to an oral disclosure, use, exhibition or other means<br>"P"   document published prior to the international filing date but later than the priority date claimed | "T"   later document published after the international filing date or priority date and not in conflict with the application but cited to understand the principle or theory underlying the invention<br>"X"   document of particular relevance; the claimed invention cannot be considered novel or cannot be considered to involve an inventive step when the document is taken alone<br>"Y"   document of particular relevance; the claimed invention cannot be considered to involve an inventive step when the document is combined with one or more other such documents, such combination being obvious to a person skilled in the art<br>"&"   document member of the same patent family |

| Date of the actual completion of the international search | Date of mailing of the international search report |
|---|---|
| **04 November 2022** | **04 November 2022** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

## INTERNATIONAL SEARCH REPORT

| International application No. |
| --- |
| **PCT/KR2022/011139** |

**C. DOCUMENTS CONSIDERED TO BE RELEVANT**

| Category* | Citation of document, with indication, where appropriate, of the relevant passages | Relevant to claim No. |
| --- | --- | --- |
| A | US 2019-0315880 A1 (GENMAB A/S) 17 October 2019 (2019-10-17)<br>See entire document. | 1-19,22-23 |

Form PCT/ISA/210 (second sheet) (July 2022)

**INTERNATIONAL SEARCH REPORT**

| International application No. |
| --- |
| **PCT/KR2022/011139** |

**Box No. I      Nucleotide and/or amino acid sequence(s) (Continuation of item 1.c of the first sheet)**

1.  With regard to any nucleotide and/or amino acid sequence disclosed in the international application, the international search was carried out on the basis of a sequence listing:

    a.  ☑  forming part of the international application as filed.

    b.  ☐  furnished subsequent to the international filing date for the purposes of international search (Rule 13*ter*.1(a)),

        ☐  accompanied by a statement to the effect that the sequence listing does not go beyond the disclosure in the international application as filed.

2.  ☐  With regard to any nucleotide and/or amino acid sequence disclosed in the international application, this report has been established to the extent that a meaningful search could be carried out without a WIPO Standard ST.26 compliant sequence listing.

3.  Additional comments:

Form PCT/ISA/210 (continuation of first sheet) (July 2022)

**INTERNATIONAL SEARCH REPORT**

| International application No. |
| --- |
| **PCT/KR2022/011139** |

| Box No. II | Observations where certain claims were found unsearchable (Continuation of item 2 of first sheet) |
| --- | --- |

This international search report has not been established in respect of certain claims under Article 17(2)(a) for the following reasons:

1. ☑ Claims Nos.: **20-21**
   because they relate to subject matter not required to be searched by this Authority, namely:

   Claims 20-21 pertain to a method for treatment of the human body by therapy (PCT Article 17(2)(a)(i) and PCT Rule 39.1(iv)).

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (July 2022)

| INTERNATIONAL SEARCH REPORT<br>Information on patent family members | | International application No.<br>**PCT/KR2022/011139** | |
|---|---|---|---|

| Patent document<br>cited in search report | Publication date<br>(day/month/year) | Patent family member(s) | Publication date<br>(day/month/year) |
|---|---|---|---|
| US 2019-0315880 A1 | 17 October 2019 | CN 103119065 A | 22 May 2013 |
| | | CN 106084053 A | 09 November 2016 |
| | | CN 111012921 A | 17 April 2020 |
| | | EP 2582728 A2 | 24 April 2013 |
| | | EP 2582728 B1 | 23 August 2017 |
| | | EP 3281956 A2 | 14 February 2018 |
| | | JP 2013-532148 A | 15 August 2013 |
| | | JP 6055404 B2 | 27 December 2016 |
| | | KR 10-1935058 B1 | 03 January 2019 |
| | | KR 10-2013-0117751 A | 28 October 2013 |
| | | US 2013-0101608 A1 | 25 April 2013 |
| | | US 2016-0067349 A1 | 10 March 2016 |
| | | US 2017-0136130 A1 | 18 May 2017 |
| | | US 2021-0395384 A1 | 23 December 2021 |
| | | US 9168314 B2 | 27 October 2015 |
| | | US 9492565 B2 | 15 November 2016 |
| | | WO 2011-157741 A2 | 22 December 2011 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20200016211 A
- JP 2016140346 A
- WO 2012093833 A
- US 20130217131 A
- US 7888121 B
- US 8409861 B
- US 6479626 B
- US 6903185 B
- US 7153949 B
- KR 101656236
- KR 101656237
- KR 101706085
- KR 102052286
- KR 102182847

**Non-patent literature cited in the description**

- **GUIDO MOLL et al.** Intravascular Mesenchymal Stromal/Stem Cell Therapy Product Diversification: Time for New Clinical Guidelines. *Trends in Molecular Medicine,* 01 February 2019, vol. 25 (2), 149-163
- **BEERLI et al.** *Nature Biotechnol.,* 2002, vol. 20, 135-141
- **PABO et al.** *Ann. Rev. Biochem.,* 2001, vol. 70, 313-340
- **ISALAN et al.** *Nature Biotechnol.,* 2001, vol. 19, 656-660
- **SEGAL et al.** *Curr. Opin. Biotechnol.,* 2001, vol. 12, 632-637
- **CHOO et al.** *Curr. Opin. Struct. Biol.,* 2000, vol. 10, 411-416
- Remington's Pharmaceutical Sciences. Mack Publishing Company, 1995